# EUROPÄISCHE PATENTSCHRIFT

(11) **EP 3 695 060 B1**
(45) Veröffentlichungstag und Bekanntmachung des Hinweises auf die Patenterteilung: **05.06.2024**
(21) Anmeldenummer: 18793183.7
(22) Anmeldetag: 09.10.2018
(51) Int. Cl.: E03B 7/04, E03B 7/02, E03B 7/08, E03C 1/02, E03D 5/10, F24D 17/00

(54) **TRINKWASSERVERSORGUNGSSYSTEM MIT DRUCKSTEUERUNG, VERFAHREN ZU DESSEN STEUERUNG SOWIE COMPUTERPROGRAMM**
DRINKING WATER SUPPLY SYSTEM WITH PRESSURE CONTROL FUNCTION, METHOD FOR CONTROLLING SAME, AND COMPUTER PROGRAM
SYSTÈME D'ALIMENTATION EN EAU POTABLE DOTÉ D'UN DISPOSITIF DE RÉGULATION DE PRESSION, PROCÉDÉ DE COMMANDE DE CE SYSTÈME ET PROGRAMME INFORMATIQUE

(30) Priorität: 09.10.2017 DE 102017123437; 13.04.2018 DE 102018108850
(43) Veröffentlichungstag der Anmeldung: 19.08.2020
(73) Patentinhaber: Viega Technology GmbH & Co. KG, 57439 Attendorn (DE)
(72) Erfinder: KRAMER, Markus, 59889 Eslohe (DE); SCHAUER, Christian, 59755 Arnsberg (DE); OTTO, Christian, 42579 Heiligenhaus (DE); STEGER, Patrick, 57233 Kreuztal (DE); LÖHER, David, 57368 Lennestadt (DE); WINTERHOLLER, Arthur, 57439 Attendorn (DE)
(74) Vertreter: Cohausz & Florack
(86) Internationale Anmeldenummer: PCT/EP2018/077414
(87) Internationale Veröffentlichungsnummer: WO 2019/072810

(56) Entgegenhaltungen:
- EP-A1- 3 214 230
- EP-A2- 2 466 019
- WO-A1-2011/053237
- DE-A1-102014 208 261
- DE-U1-202014 007 233
- US-A1- 2014 332 088

## Beschreibung

Die Erfindung betrifft ein Trinkwasserversorgungssystem mit einem Trinkwasserleitungssystem und mit mehreren an das Trinkwasserleitungssystem angeschlossenen Trinkwasserentnahmestellen. Weiterhin betrifft die Erfindung ein Verfahren zur Steuerung eines Trinkwasserversorgungssystems und ein Computerprogramm.

Trinkwasserversorgungsysteme größerer Gebäude und Einrichtungen, wie zum Beispiel eines Hotels oder Krankenhauses, sind komplexe Systeme mit einem verzweigten Trinkwasserleitungssystem und einer Vielzahl daran angeschlossener Trinkwasserentnahmestellen. Bei solchen Gebäuden bestehen zudem hohe Anforderungen an die Wasserqualität, Energieeffizient und Komfort beim Betrieb des Trinkwasserversorgungssystems.

Es hat sich herausgestellt, dass die Komplexität der Trinkwasserversorgungsysteme es in solchen Gebäuden schwierig macht, die gewünschte Wasserqualität zu jeder Zeit, an jeder Trinkwasserentnahmestelle und unabhängig von der individuellen Nutzung des Trinkwasserversorgungsystems zu gewährleisten. Weiterhin können Ausfälle einzelner Komponenten eines solchen Trinkwasserversorgungsystems unter Umständen über längere Zeit unbemerkt bleiben, wodurch die lokale Trinkwasserversorgung gestört wird oder die Wasserqualität leiden kann.

Aus der EP 2 466 019 A2 ist ein Trinkwasserversorgungssystem eines Gebäudes mit einer automatischen Spülung abhängig vom Volumenstrom und Wassertemperaturen in den Versorgungssträngen des Trinkwasserversorgungssystems bekannt. Aus der US 2014/332088 A1 ist ein System zur Vermeidung von Druckschocks in einem Leitungsnetzwerk für Flüssigkeiten bekannt, wobei bei Druckänderungen Ventile geöffnet oder geschlossen werden. Eine vom gemessenen Zuflussdruck bedingte Filterreinigung wird offenbart. Weiter ist aus der DE 10 2006 039701 B3 ein Trinkwassersystem mit druckgesteuerten Ventilen bekannt.

Vor diesem Hintergrund liegt der vorliegenden Erfindung die Aufgabe zugrunde, ein Trinkwasserversorgungssystem, ein Verfahren zu dessen Steuerung sowie ein Computerprogramm zur Verfügung zu stellen, mit denen die Trinkwasserversorgung und insbesondere die Trinkwasserqualität, Energieeffizienz und der Komfort in komplexen Trinkwasserversorgungsystemen aufrechterhalten bzw. verbessert werden kann.

Diese Aufgabe wird erfindungsgemäß gelöst durch ein Trinkwasserversorgungssystem nach Anspruch 1.

Das erfindungsgemäße Trinkwasserversorgungssystem weist ein Trinkwasserleitungssystem, mehrere an das Trinkwasserleitungssystem angeschlossene Trinkwasserentnahmestellen, mindestens einen Sensor, der dazu eingerichtet ist, Messwerte zu bestimmen, und eine zentrale Steuereinrichtung auf, die dazu eingerichtet ist, die von dem mindestens einen Sensor bestimmten Messwerte zu empfangen und auszuwerten. Das Trinkwasserleitungssystem weist einen Trinkwasserstrang zur Trinkwasserversorgung mehrerer der Trinkwasserentnahmestellen auf. Der mindestens eine Sensor ist ein Drucksensor, um den Wasserdruck im Trinkwasserstrang zu messen. Die zentrale Steuereinrichtung ist dazu eingerichtet, eine der folgenden Maßnahmen zu bewirken, wenn der vom Drucksensor gemessene Wasserdruck einen vorgegebenen Grenzwert über einen bestimmten Zeitraum unterschreitet: Beenden und/oder Unterbinden eines von der zentralen Steuereinrichtung bewirkten Ablassens von Wasser aus dem Trinkwasserstrang an einer Spüleinheit oder Beenden und/oder Unterbinden eines von der zentralen Steuereinrichtung bewirkten Abführens von Wasser aus dem Trinkwasserstrang über eine Zirkulationsleitung.

Vorzugsweise sind mehrere Sensoren vorgesehen, die dazu eingerichtet sind, an verschiedenen Stellen im Trinkwasserversorgungssystem Messwerte für eine oder verschiedene Eigenschaften des im Trinkwasserversorgungssystem geführten Wassers zu bestimmen, und die zentrale Steuereinrichtung ist dazu eingerichtet, die von den Sensoren bestimmten Messwerte zu empfangen und auszuwerten.

Weiterhin wird die oben genannte Aufgabe gelöst durch ein Verfahren nach Anspruch 10 zur Steuerung des zuvor beschriebenen Trinkwasserversorgungssystems.

Weiterhin wird die oben genannte Aufgabe erfindungsgemäß gelöst durch ein Computerprogramm, nach Anspruch 11, aufweisend Befehle, deren Ausführung auf mindestens einem Prozessor einer Steuereinrichtung des zuvor beschriebenen Trinkwasserversorgungssystems, die Durchführung des zuvor beschriebenen Verfahrens bewirkt. Insbesondere kann die zentrale Steuereinrichtung des Trinkwasserversorgungssystems einen Speicher aufweisen, auf dem das Computerprogramm gespeichert ist, wobei die Ausführung des Computerprogramms auf mindestens einem Prozessor der Steuereinrichtung die Durchführung des Verfahrens bewirkt. Die zentrale Steuereinrichtung kann auch einen Server umfassen, der Zugriff auf die zentrale Steuereinrichtung mittels eines Clients, beispielsweise über einen Webbrowser, ermöglicht, wobei die Abarbeitung des Computerprogramms Server-seitig und/oder Client-seitig erfolgen kann.

Durch das Erfassen von Messwerten, insbesondere an verschiedenen Stellen im Trinkwasserversorgungssystem für eine oder verschiedene Eigenschaften des im Trinkwasserversorgungssystem geführten Wassers, und durch die Auswertung der Messwerte in einer zentralen Steuereinrichtung ist es möglich, den Betrieb des Trinkwasserversorgungssystems an zentraler Stelle zu überwachen, sodass ein sicherer Betrieb des Gesamtsystems gewährleistet werden kann. Weiterhin ermöglicht das beschriebene Trinkwasserversorgungssystem, dass Zustände des Trinkwasserversorgungssystems, insbesondere kritische Zustände oder Fehlerzustände, die sich nur aus Messungen an einer oder insbesondere mehreren Stellen im Trinkwasserversorgungssystem ergeben, zuverlässig erkannt werden können, sodass zum Beispiel situationsangepasste Gegenmaßnahmen ergriffen werden können.

Das Trinkwasserversorgungssystem weist ein Trinkwasserleitungssystem auf. Unter dem Trinkwasserleitungssystem wird das Leitungssystem verstanden, das die Trinkwasserversorgung verschiedener Trinkwasserentnahmestellen im Trinkwasserversorgungssystem sicherstellt. Insbesondere umfasst das Trinkwasserleitungssystem zur Trinkwasserleitung geeignete Verbindungen wie Rohre, Schläuche, Verbindungsstücke, Abzweigungen etc.

An das Trinkwasserleitungssystem sind mehrere Trinkwasserentnahmestellen angeschlossen. Unter einer Trinkwasserentnahmestelle werden Komponenten verstanden, die über das Trinkwasserleitungssystem mit Trinkwasser versorgt werden und an denen Trinkwasser aus dem Trinkwasserversorgungssystem entnommen werden kann. Beispiele für Trinkwasserentnahmestellen sind zum Beispiel Wasserhähne an Waschtischen, Toiletten- oder Urinalspülungen, Wasserauslässe an Badewannen und Duschen und dergleichen.

Das Trinkwasserleitungssystem kann einen oder mehrere Trinkwasserstränge aufweisen, insbesondere einen oder mehrere Hauptversorgungsstränge, die jeweils einen oder mehrere Unterversorgungsstränge speisen, in die jeweils Trinkwasserentnahmestellen integriert sind. Handelt es sich beispielsweise um das Trinkwasserversorgungssystem eines Krankenhauses, so können zum Beispiel mehrere an einen Hauptversorgungsstrang angeschlossene Unterversorgungsstränge vorgesehen sein, die jeweils eine Station oder Einrichtung des Krankenhauses mit Trinkwasser versorgen. Ein Strang des Trinkwasserleitungssystems umfasst vorzugsweise jeweils einen Warmwasser- und einen Kaltwasserstrang. Ein Strang kann aber auch mehrere Warmwasser- und/oder Kaltwasserstränge umfassen.

Weiterhin weist das Trinkwasserversorgungssystem einen oder mehrere Sensoren auf, die vorzugsweise dazu eingerichtet sind, an verschiedenen Stellen des Trinkwasserversorgungssystems Messwerte für eine oder verschiedene Eigenschaften des im Trinkwasserversorgungssystem geführten Wassers zu bestimmen. Die Sensoren können insbesondere in das Trinkwasserleitungssystem und/oder in die Trinkwasserentnahmestellen integriert sein. Beispiele für in das Trinkwasserleitungssystem integrierte Sensoren sind zum Beispiel Druck-, Volumenstrom- oder Temperatur-Sensoren und dergleichen, die zum Beispiel in eine Leitung oder einen Fitting integriert sind. Beispiele für in eine Trinkwasserentnahmestelle integrierte Sensoren sind entsprechend Temperatur-, Druck- oder Volumenstrom-Sensoren und dergleichen, die in eine Armatur eines Waschtischs oder einer Badewanne oder Dusche oder in einen Spülkasten eines WCs oder der Zuleitung eines Urinals integriert sind.

Die Sensoren können insbesondere dazu eingerichtet sein, Messwerte für physikalische Parameter, wie zum Beispiel den Wasserdruck, die Wassertemperatur, den Volumenstrom, die Geschwindigkeitsverteilung des Wassers oder den Grad der Eintrübung durch Schwebestoffe, für chemische Parameter, wie zum Beispiel den pH-Wert, die Leitfähigkeit die Wasserhärte oder die Konzentration bestimmter Inhaltsstoffe, beispielsweise die Sauerstoffkonzentration, oder für biologische Parameter wie zum Beispiel die Keimkonzentration, insbesondere Bakterienkonzentration, im Wasser zu bestimmen.

Das Trinkwasserversorgungssystem weist weiterhin eine zentrale Steuereinrichtung auf. Die zentrale Steuereinrichtung kann beispielsweise einen Controller mit einem Mikroprozessor umfassen. Weiterhin kann die Steuereinrichtung auch mehrere verschiedene, gegebenenfalls auch voneinander entfernte und durch eine Kommunikationsverbindung miteinander verbundene Komponenten aufweisen wie zum Beispiel einen Controller und ein damit verbundenes Frontend oder einen weiteren Computer und/oder einen Server und mehrere Clients. Insbesondere kann die zentrale Steuereinrichtung selbst mit einer dezentralen Struktur aufgebaut sein, beispielsweise mit mehreren gleichberechtigten Komponenten, um die Ausfallsicherheit zu erhöhen.

Die zentrale Steuereinrichtung ist dazu eingerichtet, die von den Sensoren bestimmten Messwerte zu empfangen. Zu diesem Zweck ist die zentrale Steuereinrichtung insbesondere über Kommunikationsverbindungen mit den Sensoren verbunden. Die Sensoren können beispielweise sternförmig mit der Steuereinrichtung verbunden sein oder auch über ein Bussystem, insbesondere Feldbussystem. Neben kabelgebundenen Kommunikationsverbindungen sind auch drahtlose Kommunikationsverbindungen denkbar, die insbesondere bei einer Nachrüstung oder Erweiterung des Trinkwasserversorgungssystems vorteilhaft sind, da auf das Verlegen von Leitungen eines neuen Sensors zu einer zentralen Steuereinrichtung zumindest teilweise verzichtet werden kann.

Die zentrale Steuereinrichtung ist weiterhin dazu eingerichtet, die von dem einen oder den mehreren Sensoren bestimmten und empfangenen Messwerte auszuwerten. Insbesondere kann die Steuereinrichtung dazu eingerichtet sein, Messwerte von verschiedenen Sensoren zusammenzufassen oder Größen zu berechnen, die von den Messwerten verschiedener Sensoren abhängen.

Im Folgenden werden verschiedene Ausführungsformen des Trinkwasserversorgungssystems, des Verfahren zu dessen Steuerung und des Computerprogramms beschrieben, wobei die einzelnen Ausführungsformen jeweils separat für das Trinkwasserversorgungssystem, das Verfahren und das Computerprogramm gelten. Weiterhin sind die beschriebenen Ausführungsformen untereinander kombinierbar.

Bei einer Ausführungsform sind ein oder mehrere der Sensoren dazu eingerichtet, Messwerte für die Wassertemperatur, den Wasserdruck, den Wasserdurchfluss und/oder die Geschwindigkeitsverteilung des im Trinkwasserversorgungssystem geführten Wassers zu bestimmen. Bei einer entsprechenden Ausführungsform des Verfahrens werden Messwerte für die Wassertemperatur, den Wasserdruck, den Wasserdurchfluss und/oder die Geschwindigkeitsverteilung des im Trinkwasserversorgungssystem geführten Wassers bestimmt. Auf diese Weise können wichtige Parameter für die ordnungsgemäße Funktionsweise des Trinkwasserversorgungssystems an zentraler Stelle überwacht werden.

Durch die Überwachung der Wassertemperatur kann insbesondere überwacht werden, ob vorgegebene Temperaturgrenzwerte für die Warm- und/oder die Kaltwasserversorgung eingehalten werden. Beispielsweise schreiben bestimmte Normen eine Warmwassertemperatur von mindestens 55 °C und eine Kaltwassertemperatur von höchstens 25 °C vor. Weichen die Wassertemperaturen in einem Warm- oder Kaltwasserstrang des Trinkwasserleitungssystems hiervon ab, kann dies an zentraler Stelle festgestellt werden, so dass ggf. entsprechende Gegenmaßnahmen ergriffen werden können. Durch das Vorsehen von Temperatursensoren an verschiedenen Stellen des Trinkwasserversorgungssystems ist es zudem möglich, lokale Abweichungen von Sollvorgaben schnell zu erkennen.

Durch die Überwachung des Wasserdrucks kann insbesondere überwacht werden, ob der Druck in einem Leitungsabschnitt innerhalb vorgegebener Grenzwerte liegt. Ein zu hoher Druck kann sich nachteilig auf die Lebensdauer des Trinkwasserleitungssystems oder daran angeschlossenen Komponenten auswirken. Ein zu niedriger Druck kann dazu führen, dass einige Trinkwasserentnahmestellen nicht ausreichend versorgt werden. Durch das Vorsehen von Drucksensoren an verschiedenen Stellen des Trinkwasserversorgungssystems ist es zudem möglich, den Druckabgleich an unterschiedlichen Standorten, beispielsweise in verschiedenen Etagen, zu kontrollieren. Weiterhin kann die Überwachung des Drucks genutzt werden, um frühzeitig Leckagen im Trinkwasserversorgungsystem festzustellen. Wird zum Beispiel der Druck an verschiedenen Stellen im Trinkwasserleitungssystem über einen längeren Zeitraum überwacht und sinkt der Druck an einer Stelle plötzlich stärker ab als üblich, so kann dies auf ein Leck im entsprechenden Leitungsabschnitt hinweisen.

Durch die Überwachung des Wasserdurchflusses kann insbesondere überwacht werden, ob bestimmte Leitungsabschnitte besonders häufig oder besonders selten benutzt werden. Weiterhin können auf diese Weise Verstopfungen oder Verengungen im Leitungssystem frühzeitig erkannt werden. Darüber hinaus kann überwacht werden, ob in einem bestimmten Leitungsstrang ein ausreichender Wasseraustausch erfolgt, um zum Beispiel eine Verkeimung zu verhindern. Unter dem Wasserdurchfluss wird vorliegend die Wassermenge pro Zeit verstanden, das heißt die Wassermenge, die in einer vorgegebenen Zeit (z.B. pro Sekunde, pro Stunde oder auch pro Tag) durch den vom Wasserdurchflusssensor überwachten Leitungsabschnitt strömt.

Durch die Überwachung der Geschwindigkeitsverteilung kann insbesondere überwacht werden, ob das Wasser laminar oder turbulent durch einen bestimmten Leitungsabschnitt strömt. Zu diesem Zweck können beispielsweise in einem Leitungsabschnitt mehrere Sensoren hintereinander angeordnet werden, die die lokale Geschwindigkeit des Wassers an verschiedenen Orten des Leitungsabschnitts messen. Eine starke Schwankung der Geschwindigkeiten von Sensor zu Sensor kann zum Beispiel ein Anzeichen für eine turbulente Strömung sein. Weiterhin können Sensoren verwendet werden, die die Geschwindigkeit des Wassers an verschiedenen Positionen im Querschnitt eines Leitungsabschnitts messen.

Fließt das Wasser in einem Leitungsabschnitt turbulent, so kann dies dazu führen, dass der betreffende Leitungsabschnitt auch bei ausreichendem Wasserdurchfluss nicht vollständig gespült wird. Durch das Überwachen der Geschwindigkeitsverteilung können ein solcher Zustand erkannt und ggf. Gegenmaßnahmen ergriffen werden.

Umgekehrt kann eine turbulente Strömung für gezielte Spülvorgänge erwünscht sein, da die Wirbel einer turbulenten Strömung Ablagerungen an der Rohrwand eines Leitungsabschnitts, insbesondere einen Biofilm, abschaben können.

Bei einer Ausführungsform sind ein oder mehrere Sensoren dazu eingerichtet, Messwerte für die Trinkwassergüte des im Trinkwasserversorgungssystem geführten Wassers zu bestimmen, insbesondere für den pH-Wert, für die Sauerstoffkonzentration, für die Konzentration an freiem Chlor, für die Wasserhärte, für die Leitfähigkeit und/oder für das Vorhandensein oder die Konzentration bestimmter Inhaltstoffe wie zum Beispiel Schwebestoffe, Viren oder Mikroorganismen, insbesondere Bakterien. Bei einer entsprechenden Ausführungsform des Verfahrens werden Messwerte für die Trinkwassergüte des im Trinkwasserversorgungssystem geführten Wassers bestimmt, insbesondere für den pH-Wert, für die Sauerstoffkonzentration, für die Konzentration an freiem Chlor, für die Wasserhärte, für die Leitfähigkeit und/oder für das Vorhandensein oder die Konzentration bestimmter Inhaltstoffe wie zum Beispiel Schwebestoffe, Viren oder Mikroorganismen, insbesondere Bakterien. Auf diese Weise kann die Trinkwassergüte des Wassers direkt überwacht werden. Insbesondere kann an zentraler Stelle frühzeitig festgestellt werden, wenn das Trinkwasser zum Beispiel in einem bestimmten Leitungsabschnitt unter vorgegebene Qualitätsanforderungen sinkt, so dass Gegenmaßnahmen ergriffen werden können, bevor die Trinkwassergüte in einen für die Gesundheit kritischen Bereich gerät.

Die Trinkwassergüte (oder auch Trinkwasserqualität) wird von den Inhaltsstoffen (z.B. Schwebestoffe, chemische Inhaltsstoffe, Keime wie Viren oder Mikroorganismen) und den chemischen und biologischen Eigenschaften des Trinkwassers bestimmt. Insbesondere gibt es viele gesetzliche Grenzwerte für bestimmte Inhaltsstoffe, die bei der Trinkwasserversorgung unbedingt eingehalten werden müssen. Darüber hinaus können auch spezielle Vorgaben, zum Beispiel von speziellen Betreibern des Trinkwassersystems wie zum Beispiel Krankenhäusern und dergleichen, noch engere Grenzwerte für die Trinkwassergüte vorgeben als die gesetzlichen Grenzwerte.

Zur Messung des pH-Werts kann ein pH-Meter, beispielsweise beruhend auf dem Prinzip der Potentiometrie, verwendet werden. Zur Messung der Wasserhärte kann ein Photometer für die Wasserhärtebestimmung verwendet werden. Zur Messung der Leitfähigkeit kann ein Widerstandsmesser verwendet werden.

Die Überwachung des pH-Werts, der Wasserhärte und/oder der Leitfähigkeit ist insbesondere auch für die Lebensdauer der Systeminstallation, insbesondere des Trinkwasserleitungssystems, relevant. Eine Trinkwasserinstallation ist typischerweise für einen bestimmen pH-Bereich und eine bestimmte Wasserhärte ausgelegt und können Schaden nehmen, häufiger ausfallen bzw. eine geringere Lebensdauer aufweisen, wenn das Trinkwasser außerhalb des vorgegebenen Bereichs liegt.

Beispielsweise kommt es bei Cu-Leitungen zu einer verstärkten Korrosion, wenn das Trinkwasser zu sauer ist. Durch die Überwachung des pH-Werts bzw. der Leitfähigkeit können rechtzeitig Maßnahmen ergriffen werden, um diesem Korrosionsproblem entgegenzuwirken, beispielsweise indem das Leitungssystem bzw. einzelne Abschnitte davon gespült werden. Zusätzlich oder alternativ können Wartungsintervalle verkürzt, Cu-Rohre gegen säurebeständigere Rohre ausgetauscht und/oder Wasserbehandlungsanlagen in Betrieb genommen werden.

Ein hoher Ionen- bzw. Salzgehalt im Trinkwasser, der sich z.B. in einer erhöhten Leitfähigkeit zeigt, kann den Betrieb bestimmter Komponenten wie zum Beispiel einer ggf. vorgesehenen Enthärteranlage stören. Weiterhin kann ein hoher Härtegrad des Trinkwassers zu Kalkablagerungen führen. Durch die Überwachung der Leitfähigkeit und/oder des Härtegrads können rechtzeitig Maßnahmen ergriffen werden, um diesen Problemen entgegenzuwirken, beispielsweise indem das Leitungssystem bzw. einzelne Abschnitte davon gespült werden. Zusätzlich oder alternativ können Wartungsintervalle verkürzt und/oder Wasserbehandlungsanlagen in Betrieb genommen werden.

Zur Messung des Vorhandenseins oder die Konzentrationen bestimmter Inhaltstoffe können je nach Inhaltsstoff verschiedene Sensoren eingesetzt werden. Beispielsweise kann ein optischer Sensor eingesetzt werden, um die Trübung des Wassers durch darin enthaltene Schwebestoffe zu messen. Weiterhin kann ein Bakteriensensor verwendet werden, um einen Wert für die Anzahl der Bakterien in einem vorgegebenen Volumen Wasser, d.h. für die Bakterienkonzentration, zu bestimmen. Weiterhin können Sensoren verwendet werden, um die Konzentration bestimmter chemischer Verbindungen, beispielsweise organischer Verbindungen, im Wasser festzustellen. Zur Bestimmung einer Viren- oder Mikroorganismen-, insbesondere Bakterien-, Konzentration können beispielsweise Sensoren der Firma Roche eingesetzt werden (z.B. CEDEX- oder CASY-Analysatoren). Insbesondere können auch lab-on-a-chip-Sensoren eingesetzt werden.

Durch die Überwachung des Vorhandenseins oder der Konzentrationen bestimmter Inhaltsstoffe kann die Einhaltung vorgegebener Grenzwerte für bestimmte Inhaltsstoffe an verschiedenen Stellen im Trinkwasserversorgungssystem überwacht werden. Dadurch können Abweichungen mikrobioligischer, chemischer oder physikalischer Art frühzeitig festgestellt werden.

Insbesondere können Sensoren zur Bestimmung von Messwerten für die Trinkwassergüte auch im Bereich eines zentralen Einspeisepunkts des örtlichen Wasserversorgers in das Trinkwasserversorgungssystem vorgesehen sein. Auf diese Weise kann die Trinkwasserqualität des in das Trinkwasserversorgungssystem eingespeisten Wasser überwacht werden.

Bei einer Ausführungsform ist die Steuereinrichtung dazu eingerichtet, die Ausgabe einer von den empfangenen Messwerten abhängigen Nutzerinformation über eine Nutzerschnittschnelle zu bewirken. Bei einer entsprechenden Ausführungsform des Verfahrens wird eine von den empfangenen Messwerten abhängige Nutzerinformation ausgegeben.

Beispielsweise kann die Steuereinrichtung dazu eingerichtet sein, die von den Sensoren empfangenen Messwerte auf das Über- oder Unterschreiten eines bzw. jeweiliger Grenzwerte zu überwachen und bei Überschreitung bzw. Unterschreiten eines Grenzwerts, gegebenenfalls über einen vorgegebenen Zeitraum, eine entsprechende Warnmeldung über die Nutzerschnittstelle auszugeben.

Ein Grenzwert zur Überwachung empfangener Messwerte kann fest vorgegeben sein oder auch bestimmt werden, insbesondere durch die zentrale Steuereinrichtung. Insbesondere ist es denkbar, einen Grenzwert aus zuvor bestimmten Messwerten zu bestimmen, die beispielsweise über einen vorgegebenen Zeitraum erhoben wurden. Auf diese Weise können Abweichungen von üblichen Werten oder Wertebereichen der Vergangenheit festgestellt werden. Weiterhin ist es denkbar, Messwerte, insbesondere für die Trinkwassergüte oder allgemeiner für die Zusammensetzung des Wassers, am zentralen Einspeisepunkt des örtlichen Wasserversorgers zu bestimmen und abhängig davon einen Grenzwert zu bestimmen. Ein solcher Grenzwert kann dann insbesondere mit entsprechenden Messwerten verglichen werden, die innerhalb des Trinkwasserversorgungssystems, insbesondere an ein oder mehreren Stellen im Trinkwasserleitungssystem, bestimmt wurden. Auf diese Weise können Veränderungen des Wassers im Trinkwasserversorgungssystem, insbesondere im Trinkwasserleitungssystem überwacht werden.

Die Überwachung des Sauerstoffgehalts ermöglicht insbesondere, Korrosionsstellen im Trinkwasserversorgungssystem, insbesondere im Trinkwasserleitungssystem, frühzeitig zu erkennen. Der Korrosionsprozess entzieht dem Wasser Sauerstoff und führt dadurch zu einem Absinken des Sauerstoffgehalts. Vorzugsweise wird ein für die Sauerstoffkonzentration bestimmter Messwert auf das Unterschreiten eines Grenzwerts überwacht und bei Unterschreiten des Grenzwerts eine Warnmeldung ausgegeben, um auf eine mögliche Korrosion hinzuweisen.

Die Überwachung der Wasserhärte ermöglicht insbesondere festzustellen, ob ein im Trinkwasserversorgungssystem vorgesehener Enthärter zuverlässig arbeitet. Vorzugsweise wird ein für die Wasserhärte bestimmter Messwert auf das Überschreiten eines Grenzwerts überwacht und bei Überschreiten des Grenzwerts eine Warnmeldung ausgegeben, um auf einen möglichen Defekt des Enthärters hinzuweisen.

Die Überwachung der Konzentration an freiem Chlor ermöglicht insbesondere festzustellen, ob im Trinkwasserversorgungssystem eine Verkeimung vorliegt. Da freies Chlor mit Keimen reagiert, führt eine Verkeimung zu einer Erniedrigung der Konzentration an freiem Chlor. Vorzugsweise wird ein für die Konzentration an freiem Chlor bestimmter Messwert auf das Unterschreiten eines Grenzwerts überwacht und bei Unterschreiten des Grenzwerts eine Warnmeldung ausgegeben, um auf eine mögliche Verkeimung hinzuweisen. Der Grenzwert kann insbesondere abhängig von einem am zentralen Einspeisepunkt des örtlichen Wasserversorgers bestimmten Messwert für die Konzentration an freiem Chlor bestimmt werden. Auf diese Weise kann überwacht werden, ob die Konzentration an freiem Chlor innerhalb des Trinkwasserversorgungssystems sinkt.

Die Überwachung der Leitfähigkeit ermöglicht zum Beispiel festzustellen, in welchem Umfang eine im Trinkwasserversorgungssystem vorgesehene Opferanode, zum Beispiel eine Opferanode in einer Warmwassertherme, durch Korrosion angegriffen wird. Auf diese Weise kann zum Beispiel die voraussichtliche Lebensdauer einer Opferanode bestimmt werden oder festgestellt werden, ob eine Opferanode aufgebracht ist. Vorzugsweise wird ein für die Leitfähigkeit bestimmter Messwert auf das Überschreiten eines Grenzwerts überwacht.

Weiterhin kann die Steuereinrichtung dazu eingerichtet sein, aus den Messwerten von mehreren Sensoren eine Überwachungsgröße zu bestimmen und diese oder das Über- oder Unterschreiten der Überwachungsgröße gegenüber vorgegebenen Grenzwerten über die Nutzerschnittstelle anzuzeigen.

Bei der Nutzerschnittstelle kann es sich beispielweise um einen Bildschirm handeln. Alternativ kann das Trinkwasserversorgungssystem auch dazu eingerichtet sein, eine E-Mail oder anderweitige Textnachricht, zum Beispiel über einen Mobilfunksystem, mit einer entsprechenden Nutzerinformation zu versenden.

Bei einer weiteren Ausführungsform weist das Trinkwasserversorgungssystem mehrere dezentrale Steuerelemente auf, die dazu eingerichtet sind, an verschiedenen Stellen im Trinkwasserversorgungssystem eine oder mehrere Eigenschaften des im Trinkwasserversorgungssystem geführten Wassers zu beeinflussen, und die zentrale Steuereinrichtung ist dazu eingerichtet, die Steuerelemente zur Beeinflussung der einen oder mehreren Eigenschaften des im Trinkwasserversorgungssystem geführten Wassers anzusteuern. Bei einer entsprechenden Ausführungsform des Verfahrens werden dezentrale Steuerelemente zur Beeinflussung einer oder mehrerer Eigenschaften des im Trinkwasserversorgungssystem geführten Wassers angesteuert. Auf diese Weise kann von zentraler Stelle Einfluss auf das im Trinkwasserversorgungssystem geführte Wasser genommen werden.

Bei einer Ausführungsform sind ein oder mehrere der dezentralen Steuerelemente dazu eingerichtet, die Wassertemperatur, den Wasserdruck, den Wasserdurchfluss und/oder die Geschwindigkeitsverteilung des im Trinkwasserversorgungssystem geführten Wassers zu beeinflussen, und die zentrale Steuereinrichtung ist dazu eingerichtet, die ein oder mehreren der dezentralen Steuerelemente zur Beeinflussung der Wassertemperatur, des Wasserdrucks, des Wasserdurchflusses und/oder der Geschwindigkeitsverteilung des im Trinkwasserversorgungssystem geführten Wassers anzusteuern. Bei einer entsprechenden Ausführungsform des Verfahrens werden dezentrale Steuerelement zur Beeinflussung der Wassertemperatur, des Wasserdrucks, des Wasserdurchflusses und/oder der Geschwindigkeitsverteilung des im Trinkwasserversorgungssystem geführten Wassers angesteuert.

Als dezentrale Steuerelemente können insbesondere ansteuerbare Armaturen vorgesehen werden. Unter Armaturen werden Komponenten verstanden, mit denen Medienströme verändert, insbesondere reduziert, umgeleitet oder abgesperrt werden können. Beispiele für Armaturen sind Ventile wie Zirkulationsventile oder Regelventile.

Bei den dezentralen Steuerelementen kann es sich beispielsweise um ansteuerbare Ventile, beispielsweise Drossel- oder Regelventile, ansteuerbare Abzweigungen zur Umleitung eines Wasserflusses, Pumpen zur Beeinflussung des Wasserdrucks oder der Strömungsgeschwindigkeit des Wassers, Heiz- oder Kühlelemente zur Beeinflussung der Wassertemperatur und dergleichen handeln.

Indem die zentrale Steuereinrichtung dazu eingerichtet ist, die Steuerelemente zur Beeinflussung der Wassertemperatur, des Wasserdrucks, des Wasserdurchflusses und/oder der Geschwindigkeitsverteilung anzusteuern, ist eine zentrale Steuerung des Trinkwasserversorgungssystems möglich, sodass der Wasserfluss im gesamten Trinkwasserversorgungssystem vorzugsweise von einer Stelle gesteuert werden kann.

Bei einer weiteren Ausführungsform sind ein oder mehrere der dezentralen Steuerelemente dazu eingerichtet, die Trinkwassergüte des im Trinkwasserversorgungssystem geführten Wassers zu beeinflussen, insbesondere den pH-Wert, die Sauerstoffkonzentration, die Konzentration an freiem Chlor, die Wasserhärte, die Leitfähigkeit und/oder das Vorhandensein oder die Konzentration bestimmter Inhaltsstoffe wie zum Beispiel Schwebestoffe, Viren oder Mikroorganismen, und die zentrale Steuereinrichtung ist dazu eingerichtet, die ein oder mehreren der dezentralen Steuerelemente zur Beeinflussung der Trinkwassergüte des im Trinkwasserversorgungssystem geführten Wassers, insbesondere des pH-Werts, der Wasserhärte, der Leitfähigkeit und/oder des Vorhandenseins oder der Konzentration bestimmter Inhaltsstoffe wie zum Beispiel Schwebestoffe, Viren oder Mikroorganismen, anzusteuern. Bei einer entsprechenden Ausführungsform des Verfahrens werden dezentrale Steuerelemente zur Beeinflussung der Trinkwassergüte des im Trinkwasserversorgungssystem geführten Wassers, insbesondere des pH-Werts, der Sauerstoffkonzentration, der Konzentration an freiem Chlor, der Wasserhärte, der Leitfähigkeit und/oder des Vorhandenseins oder der Konzentration bestimmter Inhaltsstoffe wie zum Beispiel Schwebestoffe, Viren oder Mikroorganismen, angesteuert. Auf diese Weise kann die Trinkwassergüte im Trinkwasserversorgungssystem beeinflusst werden. Insbesondere kann durch die Ansteuerung der dezentralen Steuerelemente erreicht werden, dass die Trinkwassergüte im gesamten Trinkwasserversorgungssystem im gewünschten Bereich bleibt.

Zur Regulierung des pH-Werts können insbesondere Spülvorgänge durchgeführt werden, bei denen das Leitungssystem oder Abschnitte davon mit Wasser durchspült werden. Liegt der pH-Wert außerhalb eines vorgegebenen Bereichs wird weiterhin vorzugsweise ein Nutzeralarm über eine vorgesehene Nutzerschnittstelle ausgegeben, da eine derartige Änderung des pH-Werts eher ungewöhnlich ist, so dass ein solcher Vorfall sorgfältig geprüft werden sollte.

Für die Beeinflussung der Wasserhärte kann zum Beispiel ein Wasserenthärter vorgesehen sein. Zur Entfernung von Schwebstoffen oder Bakterien im Wasser können beispielsweise Filter vorgesehen sein. Weiterhin kann zur Abtötung von Bakterien eine Sterilisationsstrecke zur thermischen Behandlung des Wassers oder zur Bestrahlung des Wassers mit UV-Licht vorgesehen sein.

Der Wasserenthärter, der Filter oder die Sterilisationsstrecke können zum Beispiel in einem gesonderten Leitungsabschnitt vorgesehen sein, der über ansteuerbare Ventile mit einer Leitung des Trinkwasserleitungssystems verbunden ist. Durch das Ansteuern der Ventile kann das Wasser dann gezielt durch den gesonderten Leitungsabschnitt umgeleitet und damit durch den Wasserenthärter, den Filter oder die Sterilisationsstrecke geleitet werden.

Bei einer weiteren Ausführungsform ist die zentrale Steuereinrichtung dazu eingerichtet, eine Information über die Tageszeit zu empfangen und die Steuerelemente zur Beeinflussung der einen oder mehreren Eigenschaften des im Trinkwasserversorgungssystem geführten Wassers abhängig von der empfangenen Information über die Tageszeit anzusteuern. Bei einer entsprechenden Ausführungsform des Verfahrens werden Steuerelemente zur Beeinflussung der einen oder mehreren Eigenschaften des im Trinkwasserversorgungssystem geführten Wassers abhängig von einer Information über die Tageszeit angesteuert. Auf diese Weise kann die Steuerung des Trinkwasserversorgungssystems auf die wechselnden Anforderungen an unterschiedlichen Tageszeiten angepasst werden. Beispielsweise können verschiedene Steuerprogramme für den Tag und für die Nacht vorgesehen sein. Zum Erhalt der Information über die Tageszeit ist vorzugsweise eine Uhr, beispielsweise eine Systemuhr in der zentralen Steuereinrichtung, vorgesehen. Das Empfangen der Information über die Tageszeit erfordert demnach nicht, dass die Information von außerhalb der zentralen Steuereinrichtung zur Verfügung gestellt wird.

Bei einer weiteren Ausführungsform sind mehrere Steuerelemente zu einer virtuellen Gruppe zusammengefasst und vorzugsweise ist die Steuereinrichtung dazu eingerichtet, bei Empfang eines Befehls zur Ansteuerung der virtuellen Gruppe die einzelnen Steuerelemente der virtuellen Gruppe anzusteuern, vorzugsweise entsprechend eines für die Gruppe vordefinierten Ansteuerplans. Bei einer entsprechenden Ausführungsform des Verfahrens wird ein Befehl zur Ansteuerung einer virtuellen Gruppe, gegebenenfalls entsprechend eines vordefinierten Ansteuerplans, empfangen und die einzelnen Steuerelemente der virtuellen Gruppe werden angesteuert, insbesondere gemäß dem vordefinierten Ansteuerplan.

Auf diese Weise wird eine einfachere Steuerung des Trinkwasserversorgungssystems erreicht, da nicht mehr alle Steuerelemente einzeln angesteuert werden müssen, sondern eine gruppenweise Ansteuerung ermöglicht wird.

Weist das Trinkwasserversorgungssystem beispielsweise einem Trinkwasserstrang mit mehreren Trinkwasserentnahmestellen auf, an denen jeweils Steuerelemente zum automatischen Ablassen von Trinkwasser aus dem Trinkwasserversorgungssystem vorgesehen sind, so können diese Steuerelemente zu einer virtuellen Gruppe zusammengefasst und dann mittels eines einzelnen Befehls gemeinsam angesteuert werden. Beispielsweise kann auf diese Weise durch einen einzelnen Nutzerbefehl ein bestimmter Abschnitt des Trinkwasserversorgungssystems gespült werden.

Weiterhin können beispielsweise mehrere Drossel- und/oder Abzweigventile, die die Zufuhr eines bestimmten Trinkwasserstrangs regeln, zu einer Gruppe zusammengefasst werden, sodass diese gemeinsam geöffnet und geschlossen werden können.

Vorzugsweise ist ein Ansteuerplan definiert, der die an die einzelnen Steuerelemente der virtuellen Gruppe abzugebenden Befehle enthält. Weiterhin kann der Ansteuerplan Informationen beinhalten, zu welchen Zeitpunkten die einzelnen Steuerelemente anzusteuern sind, beispielsweise die Reihenfolge der Ansteuerung.

Das Trinkwasserversorgungssystem kann mehrere dezentrale Steuereinrichtungen, beispielsweise mehrere Controller, aufweisen, die jeweils mit ein oder mehreren dezentralen Steuerelementen zu deren Steuerung verbunden sind. Die dezentralen Steuereinrichtungen sind direkt oder indirekt mit der zentralen Steuereinrichtung verbunden, so dass die zentrale Steuereinrichtung durch Ansteuerung der jeweiligen dezentralen Steuereinrichtungen eine Steuerung der damit jeweils verbundenen dezentralen Steuerelemente bewirken kann.

Eine virtuelle Gruppe kann insbesondere dezentrale Steuerelemente umfassen, die verschiedenen dezentralen Steuereinrichtungen zugeordnet sind. Die virtuelle Gruppierung von Steuerelementen erlaubt auf diese Weise eine die einzelnen dezentralen Steuereinrichtungen übergreifende Gruppierung von Steuerelementen unabhängig von der Hardware-Architektur, d.h. von der jeweiligen Zuordnung von Steuerelementen zu bestimmten dezentralen Steuereinrichtungen.

Vorzugsweise können dezentrale Steuerelemente über die zentrale Steuereinrichtung einer virtuellen Gruppe zugeordnet werden, beispielsweise mittels einer entsprechenden Nutzereingabe über eine Nutzerschnittstelle der zentralen Steuereinrichtung. Auf diese Weise lässt sich eine virtuelle Gruppe flexibel softwareseitig einstellen, ohne dass Hardware-seitige Änderungen, wie zum Beispiel das Verbinden eines Steuerelements mit einer anderen dezentralen Steuereinrichtung, erforderlich sind.

Bei einer weiteren Ausführungsform ist ein Ansteuerplan vordefiniert, der mehrere Ansteuerbefehle für verschiedene Steuerelemente enthält und die Steuereinrichtung ist dazu eingerichtet, bei Empfang eines Befehls zur Durchführung des vordefinierten Ansteuerplans, die Steuerelemente entsprechend des Ansteuerplans anzusteuern. Bei einer entsprechenden Ausführungsform des Verfahrens ist ein Ansteuerplan vordefiniert, der mehrere Ansteuerbefehle für verschiedene Steuerelemente enthält, es wird ein Befehl zur Durchführung des vordefinierten Ansteuerplans empfangen und die Steuerelemente werden entsprechend des Ansteuerplans angesteuert.

Auf diese Weise wird die Steuerung des Trinkwasserversorgungssystems vereinfacht, indem der Nutzer durch Eingabe eines einzelnen Befehls beziehungsweise Abruf eines Ansteuerplans eine gegebenenfalls komplexe Befehlsfolge ablaufen lassen kann, die zum Beispiel verschiedene Steuerelemente zu verschiedenen Zeiten ansteuert.

Bei einer weiteren Ausführungsform weist das Trinkwasserleitungssystem einen Kaltwasserstrang zur Kaltwasserversorgung mehrerer Trinkwasserentnahmestellen auf, wobei an einem ersten Ende des Kaltwasserstrangs eine den Kaltwasserstrang mit Kaltwasser versorgende Zuleitung angeschlossen ist und wobei an einem zweiten Ende des Kaltwasserstrangs eine Zirkulationsleitung angeschlossen ist, über die Wasser aus dem Kaltwasserstrang abgeführt werden kann. Das Trinkwasserleitungssystem kann mehrere Kaltwasserstränge aufweisen, wobei einer, mehrere oder alle Kaltwasserstränge wie zuvor beschrieben an eine Zirkulationsleitung angeschlossen sein können.

Bei der Kaltwasserversorgung besteht das Problem, dass sich Verunreinigungen und Verkeimungen in einem Kaltwasserstrang bilden können, wenn die an dem Kaltwasserstrang vorgesehenen Trinkwasserentnahmestellen nicht ausreichend betätigt werden, sodass das Wasser lange im Kaltwasserstrang steht. Insbesondere kann sich das lange im Kaltwasserstrang stehende Wasser über eine bestimmte Temperatur erwärmen, wodurch eine Verkeimung (Keimwachstum) weiter begünstigt wird. Durch das Vorsehen einer Zirkulationsleitung für den Kaltwasserstrang wird erreicht, dass Kaltwasser in einem Kaltwasserstrang innerhalb des Trinkwasserversorgungssystems ausgetauscht werden kann, auch wenn die Trinkwasserentnahmestellen nicht oder nur wenig betätigt werden.

Vorzugsweise ist die Zirkulationsleitung derart angeschlossen, dass sie das darin geführte Wasser wieder zurück ins übrige Trinkwasserleitungssystem führt, sodass es anderweitig verwendet werden kann.

Bei einer weiteren Ausführungsform ist in das Trinkwasserleitungssystem ein Steuerelement integriert, vorzugsweise ein ansteuerbares Ventil, das dazu eingerichtet ist, das Abführen von Wasser aus dem Kaltwasserstrang über die Zirkulationsleitung (Zirkulieren) zu steuern. Beispielsweise kann ein solches Ventil am Übergang des Kaltwasserstrangs zur Zirkulationsleitung angeordnet sein. Durch das Vorsehen eines ansteuerbaren Ventils wird eine steuerbare Kaltwasserzirkulation ermöglicht. Durch das Öffnen des Ventils kann Wasser aus dem Kaltwasserstrang abgeleitet werden, sodass das Wasser nicht zu lange in dem Kaltwasserstrang verbleibt. Andererseits kann bei Schließen des Ventils verhindert werden, dass das Wasser unnötig zirkuliert wird oder der Druck im Trinkwasserversorgungssystem zu weit abfällt. Vorzugsweise ist die Steuereinrichtung dazu eingerichtet, das Steuerelement zu steuern.

Das Trinkwasserleitungssytem kann so eingerichtet sein, dass Wasser kontinuierlich im Kaltwasserstrang zirkuliert wird, beispielsweise indem ein ansteuerbares Ventil im Kaltwasserstrang auch im geschlossenen Zustand eine Minimalmenge Wasser passieren lässt. Auf diese Weise kann auch ein lokaler, mit den vorhandenen Sensoren nicht detektierbarer Wärmeeintrag vermieden werden.

Bei einer weiteren Ausführungsform weist das Trinkwasserleitungssystem einen Kaltwasserstrang zur Kaltwasserversorgung mehrerer Trinkwasserentnahmestellen auf und das Trinkwasserleitungssystem weist eine Kühlstrecke zur Wasserkühlung auf, die derart an den Kaltwasserstrang angeschlossen ist, um Wasser aus dem Kaltwasserstrang zu kühlen. Das Trinkwasserleitungssystem kann mehrere Kaltwasserstränge aufweisen, wobei einer, mehrere oder alle Kaltwasserstränge wie zuvor beschrieben an die Wasserkühlung angeschlossen sein können.

Es wurde festgestellt, dass die Temperatur des Wassers in einem Kaltwasserstrang bei zu langen Verweilzeiten im Kaltwasserstrang oder auch zu hohen Umgebungstemperaturen sich soweit erwärmen kann, dass eine verstärkte Verkeimung begünstigt wird. Durch das Vorsehen einer Kühlstrecke kann das unerwünscht aufgewärmte Wasser aus dem Kaltwasserstrang abgekühlt werden, sodass einer durch die Erwärmung bedingten Verkeimung entgegengewirkt werden kann.

Bei einer weiteren Ausführungsform weist die Kühlstrecke ein aktives Kühlelement, beispielsweise einen mit einem Kühlmittel betriebenen Wärmetauscher auf. Dies ermöglicht eine effiziente und schnelle Kühlung des durch die Kühlstrecke geleiteten Wassers.

Bei einer weiteren Ausführungsform weist die Kühlstrecke einen Leitungsabschnitt auf, der durch einen Bereich mit einer geringeren Durchschnittstemperatur verlegt ist als der Kaltwasserstrang. Bei der Durchschnittstemperatur kann es sich beispielsweise um die Tages-, Monats- oder Jahres-Durchschnittstemperatur handeln. Beispielsweise kann die Kühlstrecke einen Leitungsabschnitt aufweisen, der durch einen Kellerraum oder durch das Erdreich geführt wird, da diese Bereiche typischerweise eine geringere Durchschnittstemperatur aufweisen als zum Beispiel Aufenthaltsräume für Personen. Diese Ausführungsform ermöglicht eine besonders energiesparende Kühlung des Wassers, da keine aktiven Kühlaggregate erforderlich sind.

Bei einer weiteren Ausführungsform weist das Trinkwasserleitungssystem einen Kaltwasserstrang zur Kaltwasserversorgung und einen Warmwasserstrang zur Warmwasserversorgung auf und es ist eine Wärmepumpe vorgesehen, um Wärme von dem im Kaltwasserstrang geführten Wasser zu dem im Warmwasserstrang geführten Wasser zu transportieren. Die Wärmepumpe ist also dazu eingerichtet, Wärme von dem im Kaltwasserstrang geführten Wasser zu dem im Warmwasserstrang geführten Wasser zu transportieren. Dadurch werden auf ökonomische Weise gleichzeitig eine Kühlung des Wassers im Kaltwasserstrang und eine Erwärmung des Wassers im Warmwasserstrang erreicht. Die Wärmepumpe kann beispielsweise angesteuert werden, wenn die Wassertemperatur in dem Kaltwasserstrang zu hoch oder die Wassertemperatur in dem Warmwasserstrang zu niedrig ist.

Bei einer weiteren Ausführungsform weist das Trinkwasserleitungssystem ein Warmwasserleitungssystem mit einem Warmwasserhauptversorgungsstrang und mehreren davon abgehenden Warmwassernebensträngen auf, es ist eine zentrale Warmwassertherme vorgesehen, die dazu eingerichtet ist, heißes Wasser in den Warmwasserhauptversorgungsstrang einzuspeisen, und es ist eine dezentrale Warmwassertherme vorgesehen, die einem der mehreren Warmwassernebenstränge zugeordnet ist und die dazu eingerichtet ist, von dem Warmwasserhauptversorgungsstrang in den Warmwassernebenstrang, dem die dezentrale Warmwassertherme zugeordnet ist, eingeleitetes Wasser zu erhitzen.

Auf diese Weise kann Wasser in einem Warmwassernebenstrang aufgeheizt werden, ohne das Wasser in die ggf. weit entfernte zentrale Warmwassertherme zurückführen zu müssen. Ist beispielsweise in einem großen Gebäudekomplex wie einem Krankenhaus eine zentrale Warmwassertherme vorgesehen, so muss das Warmwasser von der zentralen Warmwassertherme unter Umständen einen langen Weg zurücklegen, bis es einen Warmwassernebenstrang in einem entfernten Gebäudeteil erreicht. Dadurch kann das Warmwasser, wenn es den Warmwassernebenstrang erreicht, bereits soweit abgekühlt sein, dass es nach kurzer Zeit unter eine vorgegebene Mindesttemperatur fällt, beispielsweise unter 55 °C, und zurück zur zentralen Warmwassertherme gepumpt werden müsste. Durch das Vorsehen einer dezentralen Warmwassertherme kann das Wasser wieder erhitzt werden, so dass es wieder eine ausreichende Temperatur aufweist. Die dezentrale Warmwassertherme kann beispielsweise zwischen dem Abzweig vom Warmwasserhauptversorgungsstrang zum Warmwassernebenstrang und der ersten Trinkwasserentnahmestelle des Warmwassernebenstrangs angeordnet sein.

Da die dezentrale Warmwassertherme nur die Wassermenge für den Warmwassernebenstrang erhitzen muss und, da bereits von der zentralen Warmwassertherme vorerhitztes Wasser erhitzt wird, eine kleinere Temperaturerhöhung erzielt werden muss, kann die dezentrale Warmwassertherme entsprechend kleiner und kompakter ausgelegt werden als die zentrale Warmwassertherme. Beispielsweise kann die zentrale Warmwassertherme für die Wassermenge zur Versorgung des gesamten Trinkwasserversorgungssystems und zur Erhitzung des Wassers von 20 °C auf 65 °C ausgelegt sein, während die dezentrale Warmwassertherme nur für die Wassermenge zur Versorgung des Warmwassernebenstrangs und zur Erhitzung des Wassers von 50 °C auf 65 °C ausgelegt sein muss.

Bei einer weiteren Ausführungsform weist das Trinkwasserleitungssystem einen Trinkwasserstrang auf und in den Trinkwasserstrang ist eine von der zentralen Steuereinrichtung ansteuerbare Spüleinheit integriert, durch die Wasser aus dem Trinkwasserversorgungssystem abgelassen werden kann. Bei der Spüleinheit kann es sich beispielsweise um eine ansteuerbare Trinkwasserentnahmestelle handeln, die derart ansteuerbar ist, dass Wasser aus dem Trinkwasserversorgungssystem abgelassen werden kann. Alternativ kann auch eine gesonderte Spüleinheit vorgesehen sein, deren einzige Aufgabe es ist, bei entsprechender Ansteuerung Trinkwasser aus dem Trinkwasserversorgungssystem abzulassen. Eine derartige gesonderte Spüleinheit erlaubt es, das Wasser im Trinkwasserstrang auszuwechseln, beispielsweise wenn es dort zu warm oder zu kalt geworden ist oder zu lange stand. Bei dem Trinkwasserstrang kann es sich um einen Warmwasserstrang oder um einen Kaltwasserstrang handeln.

Bei einer weiteren Ausführungsform ist eine von der zentralen Steuereinrichtung ansteuerbare dezentrale Steuereinheit vorgesehen, die dazu eingerichtet ist, einen Spülvorgang an einer Trinkwasserentnahmestelle zu bewirken, um Wasser aus dem Trinkwasserversorgungssystem abzulassen. Auf diese Weise kann eine Trinkwasserentnahmestelle, beispielsweise ein Wasserhahn an einem Waschtisch oder eine WC-Spülung, zentral angesteuert werden, um Wasser aus dem Trinkwasserversorgungssystem abzulassen.

Die zentrale Steuereinrichtung ist vorzugsweise dazu eingerichtet, durch Ansteuerung einer oder mehrerer dezentraler Steuereinheiten an mehreren Trinkwasserentnahmestellen gleichzeitig einen Spülvorgang zu bewirken, wobei die mehreren Trinkwasserentnahmestellen vorzugsweise an demselben Strang oder derselben Leitung des Trinkwasserleitungssystems angeschlossenen sind. Beispielsweise kann es sich um benachbarte Trinkwasserentnahmestellen handeln. Auf diese Weise kann während der Spülvorgänge eine höhere Fließgeschwindigkeit innerhalb des Strangs bzw. der Leitung erreicht werden, so dass es zu einer turbulenten Strömung kommt, deren Wirbel eine bessere Reinigung der Rohrwandung zum Beispiel von einem Biofilm ermöglichen.

Vorzugsweise ist die zentrale Steuereinrichtung dazu eingerichtet, die Durchführung von Spülvorgängen an einer Trinkwasserentnahmestelle abhängig von einer Information über die Tageszeit zu steuern. Auf diese Weise kann ein automatisches Spülen in der Nacht beispielsweise verhindert werden, wenn sich die betreffende Trinkwasserentnahmestelle zum Beispiel auf einer Krankenhausstation befindet, oder aber gerade während der Nacht durchgeführt werden, wenn sich die betreffende Trinkwasserentnahmestelle zum Beispiel in einem Nachts unbesetzten Bürotrakt befindet.

Bei einer weiteren Ausführungsform ist ein Akustiksensor vorgesehen, der dazu eingerichtet und/oder angeordnet ist, Messwerte für die Lautstärke zu messen, insbesondere für die Lautstärke eines oder mehrerer Spülvorgänge an einer oder mehreren Trinkwasserentnahmestellen zu messen, und vorzugsweise ist die zentrale Steuereinrichtung dazu eingerichtet, die automatische Durchführung von Spülvorgängen abhängig von den von dem Akustiksensor gemessenen Messwerten zu steuern. Auf diese Weise kann bei der automatischen Durchführung von Spülvorgängen der damit verbundene Geräuschpegel überwacht werden, so dass Spülvorgänge zum Beispiel abgebrochen oder unterbunden werden können, wenn ein vorgegebener Geräuschpegel überschritten wird. Vorzugsweise wird der vorgegebene Geräuschpegel orts- und/oder tageszeitabhängig ausgewählt.

Bei einer weiteren Ausführungsform ist ein Präsenzmelder vorgesehen, der dazu eingerichtet ist, eine Information über die Anwesenheit einer Person zu bestimmen, und vorzugsweise ist die zentrale Steuereinrichtung dazu eingerichtet, ein oder mehrere der dezentralen Steuerelemente abhängig von der Information über die Anwesenheit einer Person zu steuern. Auf diese Weise kann die Steuerung des Trinkwasserversorgungssystems davon abhängig erfolgen, ob Personen zum Beispiel im Bereich eines bestimmten Trinkwasserstrangs oder bestimmter Trinkwasserentnahmestellen präsent sind oder nicht. Stellt der Präsenzmelder beispielsweise die Präsenz von Personen fest, ist in dem bestimmten Trinkwasserstrang oder an der bestimmten Trinkwasserentnahmestelle ein anderer Bedarf an Trinkwasser zu erwarten als ohne Personen. Bei dem Präsenzmelder kann es sich zum Beispiel um einen Bewegungsmelder handeln oder auch um eine Kamera, deren Bilder mittels Personenerkennungsalgorithmen analysiert werden.

Bei einer weiteren Ausführungsform ist die zentrale Steuereinrichtung dazu eingerichtet, das Trinkwasserversorgungssystem, insbesondere die dezentralen Steuerelemente, wahlweise gemäß einem ersten vorgegebenen Programm oder gemäß einem zweiten vorgegebenen Programm zu steuern, und die zentrale Steuereinrichtung ist weiter dazu eingerichtet, abhängig von der Information über die Anwesenheit einer Person das erste oder das zweite Programm auszuwählen. Bei dem ersten Programm kann es sich beispielsweise um einen Programm für einen Normalmodus handeln und bei dem zweiten Programm um ein Abwesenheitsprogramm, beispielsweise um ein Urlaubsprogramm. Auf diese Weise kann die zentrale Steueranlage automatisch zwischen einem Normalmodus und einem Abwesenheitsmodus, beispielsweise einem Urlaubsmodus, umschalten, ohne dass der Nutzer eine entsprechende Nutzereingabe tätigen muss. Natürlich kann die zentrale Steuereinrichtung dazu eingesehen sein, abhängig von der Information über die Anwesenheit einer Person zwischen mehr als zwei Programmen auszuwählen.

Bei einer weiteren Ausführungsform ist eine von der zentralen Steuereinrichtung ansteuerbare dezentrale Steuereinheit vorgesehen ist, die dazu eingerichtet ist, einen Spülvorgang an einer Trinkwasserentnahmestelle zu bewirken, um Wasser aus dem Trinkwasserversorgungssystem abzulassen, ist der Präsenzmelder dazu eingerichtet und angeordnet, eine Information über die Anwesenheit einer Person im Bereich der Trinkwasserentnahmestelle zu bestimmen, und ist die zentrale Steuereinrichtung dazu eingerichtet, die Durchführung eines Spülvorgangs an der Trinkwasserentnahmestelle abhängig von der Information über die Anwesenheit einer Person zu steuern. Auf diese Weise kann die Sicherheit bei der automatischen Steuerung des Trinkwasserversorgungssystems verbessert werden. Insbesondere kann eine automatische Spülung an einer Trinkwasserentnahmestelle abgebrochen oder unterbunden werden, wenn festgestellt wird, dass sich eine Person im Bereich der Trinkwasserentnahmestelle befindet. Dadurch kann verhindert werden, dass die betreffende Person unverhofft durch abgelassenes Wasser nass wird oder ggf. sogar bei Ablassen von heißem Wasser verbrüht wird.

Bei einer weiteren Ausführungsform weist das Trinkwasserleitungssystem einen Trinkwasserstrang auf, ist eine Gruppe von Trinkwasserentnahmestellen vorgesehen, die an dem Trinkwasserstrang angeschlossen sind, sind mehrere dezentrale Sensoren vorgesehen sind, die dazu eingerichtet sind, Informationen über die Durchführung von Spülvorgängen an Trinkwasserentnahmestellen der Gruppe von Trinkwasserentnahmestellen zu bestimmen, und ist die zentrale Steuereinrichtung dazu eingerichtet, abhängig von den Informationen über die Durchführung von Spülvorgängen an Trinkwasserentnahmestellen der Gruppe von Trinkwasserentnahmestellen die Durchführung von Spülvorgängen an einzelnen Trinkwasserentnahmestellen der Gruppe von Trinkwasserentnahmestellen zu steuern.

Die für die Durchführung von Spülvorgängen an den einzelnen Trinkwasserentnahmestellen vorgesehenen Steuerelemente können insbesondere zu einer virtuellen Gruppe zusammengefasst sein.

Auf diese Weise kann durch die zentrale Steuereinrichtung für einen gesamten Trinkwasserstrang mit mehreren Trinkwasserentnahmestellen überwacht werden, ob der Trinkwasserstrang durch Betätigung einer der Trinkwasserentnahmestellen ausreichend gespült wird und, wenn dies nicht der Fall ist, durch eine automatische Spülung an einzelnen der Trinkwasserentnahmestellen eine entsprechende Spülung herbeigeführt werden. Gegenüber autark gesteuerten Trinkwasserentnahmestellen, die automatisch Spülvorgänge durchführen, wenn sie über eine vorgegebene Zeit nicht betätigt wurden, hat dies den Vorteil, dass die zentrale Steuereinrichtung die Gesamtheit der Trinkwasserentnahmestellen überwacht, so dass eine automatische Spülung an einer Trinkwasserentnahmestelle unterbleiben kann, wenn zum Beispiel kürzlich eine benachbarte Trinkwasserentnahmestelle gespült wurde. Weiterhin können, wenn eine Spülung erforderlich ist, einzelne Trinkwasserentnahmestellen anstelle von allen Trinkwasserentnahmestellen angesteuert werden. Hierdurch wird die Spülmenge und die Häufigkeit von Spülungen reduziert, so dass Wasser eingespart werden kann.

Bei einer weiteren Ausführungsform sind ein oder mehrere dezentrale Sensoren vorgesehen, die dazu eingerichtet sind, Informationen über die Durchführung von Spülvorgängen in einem vorgegebenen Abschnitt des Trinkwasserleitungssystems zu bestimmen, und ist die zentrale Steuereinrichtung dazu eingerichtet, die Zeitdauer seit der letzten Spülung in dem vorgegebenen Abschnitt des Trinkwasserleitungssystems zu überwachen und bei Überschreiten einer vorgegebenen Maximalzeitdauer eine Spülung in dem vorgegebenen Abschnitt des Trinkwasserleitungssystems zu veranlassen. Auf diese Weise kann zentral überwacht werden, ob ein Abschnitt des Trinkwasserleitungssystems häufig genug gespült wird und, falls dies nicht der Fall ist, automatisch gespült werden.

Bei einer weiteren Ausführungsform ist die zentrale Steuereinrichtung dazu eingerichtet, die Steuerelemente abhängig von den empfangenen Messwerten zu steuern. Auf diese Weise wird eine Regelung des Trinkwasserversorgungssystems erreicht. Dadurch kann beispielsweise ein dauerhafter Betrieb in einem sicheren Parameterfenster erreicht werden, etwa indem Abweichungen aus dem Sollbereich automatisch entgegengewirkt wird.

Bei einer weiteren Ausführungsform weist das Trinkwasserleitungssystem einen Kaltwasserstrang zur Kaltwasserversorgung mehrerer Trinkwasserentnahmestellen auf, es ist ein Temperatursensor vorgesehen, um die Wassertemperatur des Wassers im Kaltwasserstrang zu messen, und die Steuereinrichtung ist dazu eingerichtet, eine Maßnahme zu bewirken, wenn die vom Temperatursensor gemessene Temperatur einen vorgegebenen Grenzwert überschreitet. Um die Wassertemperatur des Wassers im Kaltwasserstrang zu messen, kann der Temperatursensor insbesondere in den Kaltwasserstrang oder in eine an den Kaltwasserstrang angeschlossene Trinkwasserentnahmestelle integriert sein. Bei der entsprechenden Ausführungsform des Verfahrens wird eine Maßnahme bewirkt, wenn ein von einem Temperatursensor in einem Kaltwasserstrang gemessene Temperatur einen Grenzwert überschreitet.

Bei der Maßnahme kann es sich insbesondere um das Kühlen des Wassers aus dem Kaltwasserstrang in einer Kühlstrecke handeln. Weiterhin kann es sich bei der Maßnahme um das Ablassen des Wassers aus dem Kaltwasserstrang an einer Spüleinheit handeln. Weiterhin kann es sich bei der Maßnahme um das Abführen des Wassers aus dem Kaltwasserstrang über eine Zirkulationsleitung handeln.

Durch das Bewirken einer oder mehrerer dieser Maßnahmen wird der erhöhten Temperatur im Kaltwasserstrang entgegengewirkt, sodass eine drohende Verkeimung verhindert werden kann. Durch das Kühlen des Wassers wird die Temperatur des Wassers wieder gesenkt, sodass eine Keimbildung wirksam verhindert wird. Durch das Ablassen des Wassers an einer Spüleinheit wird das zu verkeimen drohende Wasser aus dem Trinkwasserversorgungssystem abgelassen, sodass frisches Wasser in den Kaltwasserstrang nachströmen kann. Durch das Abführen des Wassers über eine Zirkulationsleitung wird das Wasser aus dem Kaltwasserstrang abgeführt, sodass nachströmendes frisches Wasser in den Kaltwasserstrang gelangt. Anders als beim Ablassen des Wassers an einer Spüleinheit verbleibt das über die Zirkulationsleitung abgeführte Wasser vorzugsweise im Trinkwasserversorgungssystem und kann an anderer Stelle weiterverwendet werden.

Das Trinkwasserleitungssystem kann so eingerichtet sein, dass das aus dem Kaltwasserstrang abgeführte Trinkwasser zu einer Warmwassertherme zur Warmwasserversorgung geführt wird, beispielsweise mittels eines ansteuerbaren Dreiwegeventils, insbesondere wenn entsprechend der zuvor beschriebenen Ausführungsform die von einem Temperatursensor zur Messung der Temperatur im Kaltwasserstrang gemessene Temperatur einen vorgegebenen Grenzwert überschreitet. Auf diese Weise kann das Wasser aus dem Kaltwasserstrang, das aufgrund einer Erwärmung zum Beispiel einen erhöhten Bakteriengehalt aufweisen könnte, innerhalb des Systems sicher weiterverwendet werden, da die Bakterien durch die Wärmebehandlung in der Warmwassertherme abgetötet werden. Alternativ kann das aus dem Kaltwasserstrang abgeführte Trinkwasser grundsätzlich zur Warmwassertherme geführt werden.

Weiterhin kann es sich bei der Maßnahme um die Ausgabe einer Nutzermeldung handeln. Durch die Ausgabe einer Nutzermeldung kann beispielsweise eine verantwortliche Person auf eine drohende Verkeimung hingewiesen werden.

Bei einer weiteren Ausführungsform weist das Trinkwasserleitungssystem einen Warmwasserstrang zur Warmwasserversorgung mehrerer Trinkwasserentnahmestellen auf, es ist ein Temperatursensor vorgesehen, um die Wassertemperatur des Wassers im Warmwasserstrang zu messen, und die Steuereinrichtung ist dazu eingerichtet, eine Maßnahme zu bewirken, wenn die vom Temperatursensor gemessene Temperatur einen vorgegebenen Grenzwert unterschreitet. Um die Wassertemperatur des Wassers im Warmwasserstrang zu messen, kann der Temperatursensor insbesondere in den Warmwasserstrang oder in eine an den Warmwasserstrang angeschlossene Trinkwasserentnahmestelle integriert sein. Bei einer entsprechenden Ausführungsform des Verfahrens wird eine Maßnahme bewirkt, wenn die von einem Temperatursensor in einem Warmwasserstrang gemessene Temperatur einen vorgegebenen Grenzwert unterschreitet.

Bei der Maßnahme kann es sich um das Ablassen des Wassers aus dem Warmwasserstrang an einer Spüleinheit handeln. Das Ablassen kann natürlich auch an mehreren Spüleinheiten erfolgen. Weiterhin kann es sich bei der Maßnahme um das Abführen des Wassers aus dem Warmwasserstrang über eine Zirkulationsleitung handeln. Weiterhin kann es sich bei der Maßnahme um die Ausgabe einer Nutzermeldung handeln.

Sinkt die Temperatur in einem Warmwasserstrang zu sehr ab, so kann es zu einer erhöhten Keimbildung kommen, da die Wassertemperatur nicht mehr ausreicht, um Bakterien und dergleichen abzutöten.

Durch das Ablassen des Wassers aus dem Warmwasserstrang wird dieses aus dem Trinkwasserversorgungssystem entfernt, sodass frisches, ausreichend warmes Wasser in den Warmwasserstrang nachströmen kann. Entsprechend wird beim Abführen des Wassers aus dem Warmwasserstrang über eine Zirkulationsleitung das Wasser aus dem Warmwasserstrang abgeführt, sodass entsprechend heißes Wasser nachströmen kann. Das Wasser bleibt beim Abführen über eine Zirkulationsleistung vorzugsweise weiter im Trinkwasserversorgungssystem verfügbar und kann daher an anderer Stelle weiterverwendet werden, beispielsweise erneut aufgeheizt werden in einer entsprechend vorgesehenen Therme. Durch die Ausgabe einer Nutzermeldung kann beispielsweise eine verantwortliche Person auf eine drohende Verkeimung hingewiesen werden.

Bei einer weiteren Ausführungsform weist das Trinkwasserleitungssystem einen Trinkwasserstrang zur Trinkwasserversorgung mehrerer Trinkwasserentnahmestellen auf, es ist ein Volumenstromsensor vorgesehen, um den Volumenstrom des Wassers im Trinkwasserstrang zu messen, und die Steuereinrichtung ist dazu eingerichtet, abhängig von den vom Volumenstromsensor gemessenen Volumenstrom einen Wert für das Wasservolumen zu bestimmen, das innerhalb einer vorgegebenen Zeitdauer durch den Volumenstromsensor geflossen ist, und eine Maßnahme zu bewirken, wenn der Wert für das Wasservolumen einen vorgegebenen Grenzwert unterschreitet. Um den Volumenstrom des Wassers im Trinkwasserstrang zu messen, kann der Volumenstromsensor insbesondere in den Trinkwasserstrang integriert sein. Bei einer entsprechenden Ausführungsform des Verfahrens wird abhängig von einem von einem Volumenstromsensor in einem Trinkwasserstrang gemessenen Volumenstrom ein Wert für das Wasservolumen bestimmt, das innerhalb einer vorgegebenen Zeitdauer durch den Volumenstromsensor geflossen ist, und eine Maßnahme wird bewirkt, wenn der Wert für das Wasservolumen einen vorgegebenen Grenzwert unterschreitet.

Bei der Maßnahme kann es sich insbesondere um das Ablassen des Wassers aus dem Trinkwasserstrang an einer Spüleinheit handeln. Das Ablassen kann natürlich auch an mehreren Spüleinheiten erfolgen. Weiterhin kann es sich bei der Maßnahme um das Abführen des Wassers aus dem Trinkwasserstrang über eine Zirkulationsleitung handeln. Weiterhin kann es sich bei der Maßnahme auch um die Ausgabe einer Nutzermeldung handeln.

Das Unterschreiten des durch den Volumenstromsensor geflossenen Volumens unter einen Grenzwert zeigt an, dass gegebenenfalls zu wenig Wasser aus dem Trinkwasserstrang entnommen wird und das Trinkwasser daher gegebenenfalls zu lange im Trinkwasserstrang steht. Durch das Ablassen beziehungsweise Abführen des Wassers aus dem Trinkwasserstrang kann der Volumenaustausch automatisch bewirkt werden, sodass frisches Wasser in den Trinkwasserstrang nachströmen kann.

Weiterhin kann ein zu geringer Volumenstrom auch darauf hindeuten, dass eine bestimmte Trinkwasserentnahmestelle oder Gruppe von Trinkwasserentnahmestellen weniger genutzt werden als andere Trinkwasserentnahmestellen. Dies kann beispielsweise auf einen Defekt der entsprechenden Trinkwasserentnahmestelle, zum Beispiel ein defektes WC, hindeuten. Durch die Ausgabe einer Nutzerausgabe kann dann beispielsweise ein Hausmeister darauf aufmerksam gemacht werden, dass ein bestimmtes WC nicht genutzt wird, sodass er nachschauen kann, ob hier gegebenenfalls Reparaturbedarf besteht.

Der vorgegebene Grenzwert kann beispielsweise abhängig von den Werten von Volumenstromsensoren an anderen Trinkwassersträngen beziehungsweise Trinkwasserentnahmestellen berechnet werden. Auf diese Weise kann ermittelt werden, wenn die Entnahme in einem bestimmten Bereich eines Trinkwasserstrangs beziehungsweise an einer bestimmten Trinkwasserentnahmestelle ungewöhnlich hoch oder ungewöhnlich niedrig ist, sodass entsprechende Maßnahmen getroffen werden können.

Bei einer weiteren Ausführungsform weist das Trinkwasserleitungssystem einen Trinkwasserstrang zur Trinkwasserversorgung mehrerer Trinkwasserentnahmestellen auf, es ist ein Volumenstromsensor vorgesehen, um den Volumenstrom des Wassers im Trinkwasserstrang zu messen, und die Steuereinrichtung ist dazu eingerichtet, eine Maßnahme zu bewirken, wenn der vom Volumenstromsensor gemessene Volumenstrom einen vorgegebenen Grenzwert überschreitet. Um den Volumenstrom des Wassers im Trinkwasserstrang zu messen, kann der Volumenstromsensor insbesondere in den Trinkwasserstrang integriert sein. Bei einer entsprechenden Ausführungsform des Verfahrens wird eine Maßnahme bewirkt, wenn der von einem Volumenstromsensor in einem Trinkwasserstrang gemessene Volumenstrom einen vorgegebenen Grenzwert überschreitet.

Bei der Maßnahme kann es sich um insbesondere um die Erhöhung des Wasserdrucks handeln, beispielsweise durch Aktivierung oder Leistungserhöhung einer im Trinkwasserversorgungssystem vorgesehenen Wasserpumpe zur Durchfluss- und/oder Druckerhöhung im Trinkwasserstrang oder durch Öffnen eines Zuleitungsventils, durch das dem betroffenen Trinkwasserstrang mehr Wasser zugeführt wird. Weiterhin kann es sich bei der Maßnahme um das Beenden und/oder Unterbinden eines von der zentralen Steuereinrichtung bewirkten Ablassens von Wasser aus dem Trinkwasserstrang an einer Spüleinheit handeln. Weiterhin kann es sich bei der Maßnahme um ein Beenden und/oder Unterbinden eines von der zentralen Steuereinrichtung bewirkten Abführens von Wasser aus dem Trinkwasserstrang über eine Zirkulationsleitung handeln.

Das Überschreiten eines vorgegebenen Volumenstroms zeigt an, dass der Trinkwasserstrang stärker genutzt wird, als seine Kapazität es erlaubt, beispielsweise weil an zu vielen Trinkwasserentnahmestellen gleichzeitig Wasser entnommen wird. Dies kann dazu führen, dass der Wasserstrom oder der Druck an den einzelnen Wasserentnahmestellen abnimmt, sodass die Trinkwasserentnahmestellen gegebenenfalls nicht mehr richtig benutzt werden können.

Durch das Erhöhen des Wasserdrucks, insbesondere das Aktivieren oder die Leistungserhöhung einer im Trinkwasserversorgungssystem vorgesehenen Wasserpumpe bzw. das Öffnen eines Zuleitungsventils, kann der Durchfluss beziehungsweise der Druck im Trinkwasserstrang erhöht werden, sodass auch bei einer stärkeren Nutzung des Trinkwasserstrangs die Wasserversorgung gewährleistet bleibt. Insbesondere kann die Druck- oder Durchflusserhöhung auf den Zeitraum begrenzt werden, in dem die Überlastung des Trinkwasserstrangs vorliegt, beispielsweise um Strom zu sparen oder die mechanische Belastung des Trinkwasserstrangs zu reduzieren.

Durch das Beenden und/oder Unterbinden eines von der zentralen Steuereinrichtung bewirkten Ablassens beziehungsweise Abführens von Wasser aus dem Trinkwasserstrang kann verhindert werden, dass durch eine derartige zentral gesteuerte Entnahme von Wasser aus dem Trinkwasserstrang der Leitungsdruck oder das die zur Wasserversorgung zur Verfügung stehende Wassermenge weiter reduziert wird, sodass mehr Wasser beziehungsweise ein höherer Druck für die übrigen Trinkwasserentnahmestellen verbleibt.

Weiterhin kann es sich bei der Maßnahme um die Ausgabe einer Nutzermeldung handeln. Durch die Ausgabe einer Nutzermeldung kann eine verantwortliche Person zum Beispiel auf einen möglichen Engpass bei der Trinkwasserversorgung hingewiesen werden.

Erfolgt, während Wasser von der zentralen Steuereinrichtung gesteuert über eine Zirkulationsleitung aus einem Trinkwasserstrang abgeführt wird, eine plötzliche Volumenstromerhöhung durch das Aktivieren weiterer Trinkwasserentnahmestellen, beispielsweise durch das Öffnen mehrerer Wasserhähne, so wird vorzugsweise die von der zentralen Steuereinrichtung gesteuert Zirkulation automatisch unterbrochen.

Die Maßnahme kann von der Steuereinrichtung vorzugsweise bewirkt werden, wenn der vom Volumenstromsensor gemessene Volumenstrom über einen vorgegebenen Zeitraum oberhalb eines vorgegebenen Grenzwerts liegt. Auf diese Weise kann verhindert werden, dass bei sehr kurzfristigen Volumenstromänderungen eine ggf. unnötige Gegenmaßnahme ergriffen wird.

Erfindungsgemäß weist das Trinkwasserleitungssystem einen Trinkwasserstrang zur Trinkwasserversorgung mehrerer der Trinkwasserentnahmestellen auf, ist ein Drucksensor vorgesehen, um den Wasserdruck im Trinkwasserstrang zu messen, und die Steuereinrichtung ist dazu eingerichtet, eine Maßnahme zu bewirken, wenn der vom Drucksensor gemessene Wasserdruck einen vorgegebenen Grenzwert unterschreitet. Um den Wasserdruck im Trinkwasserstrang zu messen, kann der Drucksensor insbesondere in den Trinkwasserstrang oder in eine an den Trinkwasserstrang angeschlossene Trinkwasserentnahmestelle integriert sein. Bei einer entsprechenden Ausführungsform des Verfahrens wird eine Maßnahme bewirkt, wenn der von einem Drucksensor in einem Trinkwasserstrang gemessene Wasserdruck einen vorgegebenen Grenzwert unterschreitet.

Bei der Maßnahme kann es sich beispielsweise um ein Erhöhen des Wasserdrucks, insbesondere durch die Aktivierung oder Leistungserhöhung einer im Trinkwasserversorgungssystem vorgesehenen Wasserpumpe zur Durchfluss- und/oder Druckerhöhung im Trinkwasserstrang oder durch Öffnen eines Zuleitungsventils, handeln. Erfindungsgemäß handelt es sich bei der Maßnahme um das Beenden und/oder Unterbinden eines von der zentralen Steuereinrichtung bewirkten Ablassens von Wasser aus dem Trinkwasserstrang an einer Spüleinheit oder um das Beenden und/oder Unterbinden eines von der zentralen Steuereinrichtung bewirkten Abführens von Wasser aus dem Trinkwasserstrang über eine Zirkulationsleitung.

Wie bei der zuvor beschriebenen Ausführungsformen kann auch ein Unterschreiten eines Drucks unter einen Mindestwert ein Zeichen dafür sein, dass ein Trinkwasserstrang überlastet wird. Durch die beschriebenen Maßnahmen kann erreicht werden, dass die Überlastung des Trinkwasserstrangs während des Belastungszeitraums aufgehoben wird.

Weiterhin kann es sich bei der Maßnahme um die Ausgabe einer Nutzermeldung handeln. Durch die Ausgabe einer Nutzermeldung kann eine verantwortliche Person zum Beispiel auf einen möglichen Engpass bei der Trinkwasserversorgung hingewiesen werden.

Erfindungsgemäß wird die Maßnahme bewirkt, wenn der vom Drucksensor gemessene Wasserdruck den vorgegebenen Grenzwert über einen bestimmten Zeitraum unterschreitet, um bei kurzfristigen Druckschwankungen gegebenenfalls unnötige Gegenmaßnahmen zu vermeiden.

Bei einer weiteren Ausführungsform weist das Trinkwasserleitungssystem einen Trinkwasserstrang zur Trinkwasserversorgung mehrerer Trinkwasserentnahmestellen auf, es ist ein Drucksensor vorgesehen, um den Wasserdruck im Trinkwasserstrang zu messen, und die Steuereinrichtung ist dazu eingerichtet, eine Maßnahme zu bewirken, wenn der vom Drucksensor gemessene Wasserdruck einen vorgegebenen Grenzwert überschreitet. Um den Wasserdruck im im Trinkwasserstrang zu messen, kann der Drucksensor insbesondere in den Trinkwasserstrang oder in eine an den Trinkwasserstrang angeschlossene Trinkwasserentnahmestelle integriert sein. Bei einer entsprechenden Ausführungsform des Verfahrens wird eine Maßnahme bewirkt, wenn der vom Drucksensor in einem Trinkwasserstrang gemessene Wasserdruck einen vorgegebenen Grenzwert überschreitet.

Bei der Maßnahme kann es sich beispielsweise um eine Reduzierung des Wasserdrucks handeln, insbesondere durch die Deaktivierung oder Leistungsreduzierung einer im Trinkwasserversorgungssystem vorgesehenen Wasserpumpe zur Durchfluss- und/oder Druckerhöhung im Trinkwasserstrang oder durch Schließen eines Zuleitungsventils. Weiterhin kann es sich bei der Maßnahme um das Ablassen des Wassers aus dem Kaltwasserstrang an einer Spüleinheit handeln. Das Ablassen kann natürlich auch an mehreren Spüleinheiten erfolgen.

Werden mehrere Trinkwasserentnahmestellen gleichzeitig geschlossen, so kann es zu einer Druckerhöhung im Trinkwasserstrang kommen. Dieser kann durch die zuvor beschriebenen Maßnahmen entgegengewirkt werden. Auf diese Weise kann die mechanische Belastung des Trinkwasserleitungssystems reduziert und damit eine längere Lebensdauer erreicht werden.

Weiterhin kann es sich bei der Maßnahme um die Ausgabe einer Nutzermeldung handeln. Auf diese Weise kann eine für den sicheren Betrieb des Trinkwasserversorgungssystems verantwortliche Person über einen ggf. kritischen Überdruck hingewiesen werden.

Bei einer weiteren Ausführungsform ist die Steuereinrichtung dazu eingerichtet, eine Steuerung des Trinkwasserversorgungssystems gemäß dem zuvor beschriebenen Verfahren oder einer Ausführungsform davon zu bewirken. Beispielsweise kann die Steuereinrichtung einen Speicher mit Befehlen aufweisen, deren Ausführung auf mindestens einen Prozessor der Steuereinrichtung die Durchführung des zuvor beschriebenen Verfahrens bewirkt.

Weitere Merkmale und Vorteile der vorliegenden Erfindung ergeben sich aus der nachfolgenden Beschreibung von Ausführungsbeispielen, wobei auf die beigefügte Zeichnung Bezug genommen wird.

In der Zeichnung zeigen
- Fig. 1a-b: einen Ausschnitt eines ersten Ausführungsbeispiels des Trinkwasserversorgungssystems,
- Fig. 2: ein Ausführungsbeispiel der zentralen Steuereinrichtung des Trinkwasserversorgungssystems aus Fig. 1a,
- Fig. 3a-d: vier Ausführungsbeispiele für Kühlstrecken für das Trinkwasserversorgungssystem aus Fig. 1a,
- Fig. 4: einen weiteren Ausschnitt des Trinkwasserversorgungssystems aus Fig. 1a,
- Fig. 5: eine Wärmepumpe für das Trinkwasserversorgungssystem aus Fig. 1a,
- Fig. 6a-b: zwei Ausführungsbeispiele für Steuerelemente für das Trinkwasserversorgungssystem aus Fig. 1a,
- Fig. 7: einen weiteren Ausschnitt des Trinkwasserversorgungssystems aus Fig. 1a,
- Fig. 8: ein weteres Ausführungsbeispiel des Trinkwasserversorgungssystems,
- Fig. 9: ein Ausführungsbeispiel des Verfahrens zur Überwachung und Regelung der Kaltwassertemperatur,
- Fig. 10: ein weiteres Ausführungsbeispiel des Verfahrens zur Überwachung und Regelung der Warmwassertemperatur,
- Fig. 11: ein weiteres Ausführungsbeispiel des Verfahrens zur Überwachung und Regelung des Mindestdurchsatzes,
- Fig. 12: ein weiteres Ausführungsbeispiel des Verfahrens zur Überwachung und nutzungsabhängigen Regelung,
- Fig. 13: ein weiteres Ausführungsbeispiel des Verfahrens zur Überwachung und Regelung des Leitungsdrucks und
- Fig. 14: ein weiteres Ausführungsbeispiel des Verfahrens zur Überwachung und Regelung des Leitungsdrucks.

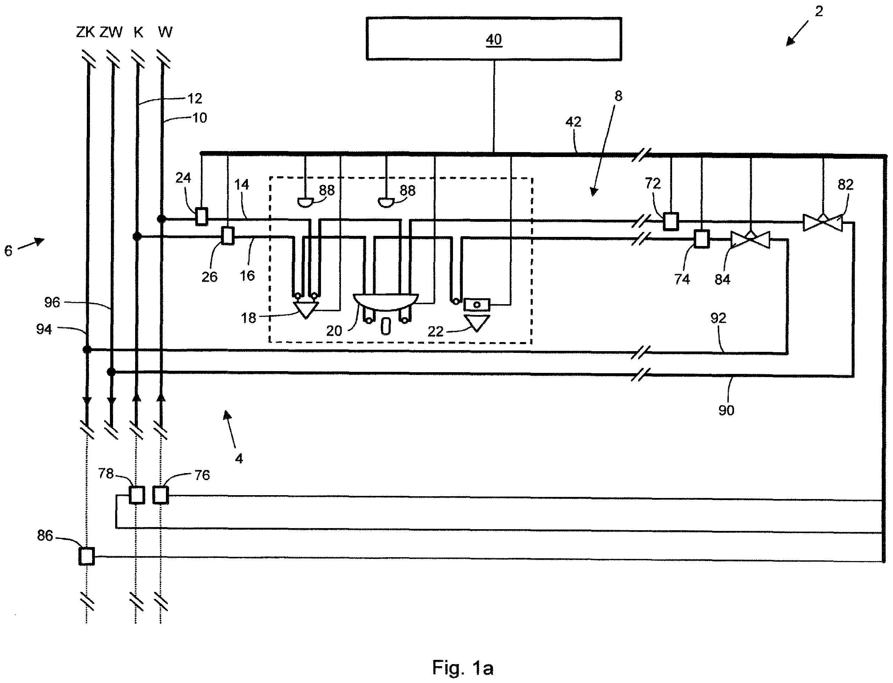

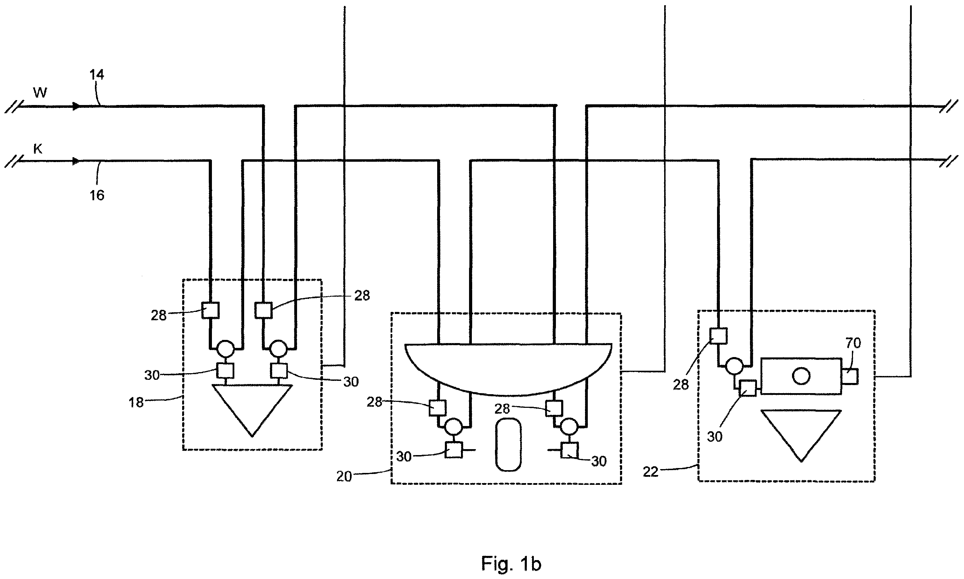

Fig. 1a zeigt ein erstes Ausführungsbeispiel des Trinkwasserversorgungssystems 2 in schematischer Darstellung. Fig. 1b zeigt den in Fig. 1a mit einer gestrichelten Linie umrandeten Ausschnitt des Trinkwasserversorgungssystems 2 in vergrößerter Darstellung.

Das Trinkwasserversorgungssystem 2 umfasst ein Trinkwasserleitungssystem 4 mit einem Hauptversorgungsstrang 6 und mehreren Unterversorgungssträngen, von denen in Figur 1 ein Unterversorgungsstrang 8 dargestellt ist. Der Hauptversorgungsstrang 6 weist eine Warmwasserzuleitung 10 ("W" in Fig. 1a) und eine Kaltwasserzuleitung 12 ("K" in Fig. 1a) auf, an die ein jeweiliger Warmwasserstrang 14 und Kaltwasserstrang 16 des Unterversorgungsstrangs 8 angeschlossen sind.

An den Warm- und Kaltwasserstrang 14,16 des Unterversorgungsstrangs 8 sind verschiedene Trinkwasserentnahmestellen angeschlossen. Figur 1 zeigt exemplarisch drei Trinkwasserentnahmestellen einer einzelnen Nasszelle mit einer ersten Trinkwasserentnahmestelle 18 als Duscharmatur mit Warm- und Kaltwasseranschluss, einer zweiten Trinkwasserentnahmestelle 20 als Waschtischarmatur mit Kalt- und Warmwasseranschluss und einer dritten Trinkwasserentnahmestelle 22 als WC-Spülung mit Kaltwasseranschluss. An den Unterversorgungsstrang 8 können eine Vielzahl weiterer Trinkwasserentnahmestellen angeschlossen sein, beispielsweise sämtliche Nasszellen einer Krankenhausstation, eine große Duschen- oder Toilettenanlage oder auch die Trinkwasserentnahmestellen eines OP-Bereichs.

An das Trinkwasserleitungssystem 4 kann eine Vielzahl weiterer Trinkwasserentnahmestellen angeschlossen sein. Beispielsweise kann es sich bei dem Trinkwasserleitungssystem 4 um das Trinkwasserleitungssystem eines Krankenhauses mit mehreren Bauabschnitten oder Etagen handeln, wobei die einzelnen Etagen, Bauabschnitte oder Stationen des Krankenhauses jeweils durch einen oder mehrere Unterversorgungsstränge versorgt werden, die wiederum über den Hauptversorgungstrang 6 gespeist werden. Sofern erforderlich, können auch mehrere Hauptversorgungsstränge vorgesehen sein, die beispielsweise einzelne Bauabschnitte des Krankenhauses versorgen.

An einen Unterversorgungsstrang 8 können beispielsweise alle Nasszellen einer Krankenhausstation, ein WC- oder Duschtrakt oder auch verschiedene Trinkwasserentnahmestellen eines OP-Bereichs angeschlossen sein.

Insgesamt zeigt Fig. 1a damit nur einen Teil eines gesamten Trinkwasserversorgungssystems 2, das einen oder mehrere Hauptversorgungsstränge und mehrere Unterversorgungsstränge mit einer Vielzahl von Trinkwasserentnahmestellen aufweisen kann.

Bei einem derart komplexen System mit einer Vielzahl von Leitungen und Trinkwasserentnahmestellen besteht das Problem, dass ein Fehler in dem Trinkwasserversorgungssystem unter Umständen unbemerkt bleibt oder nicht ohne weiteres lokalisiert werden kann. Dadurch kann es zu Teil- oder Gesamtausfällen der Trinkwasserversorgung und auch zu einer Verkeimung des Trinkwassers kommen. Insbesondere kann eine mangelnde Kontrolle beziehungsweise Wartung des Trinkwasserversorgungssystems dazu führen, dass die gewünschte Trinkwasserqualität an den einzelnen Trinkwasserentnahmestellen nicht kontinuierlich erreicht werden kann.

Um dieses Problem zu überwinden sind im Trinkwasserversorgungssystem 2 eine Vielzahl von Sensoren vorgesehen, die an verschiedenen Stellen im Trinkwasserversorgungssystem 2 Messwerte bestimmen, insbesondere für die Wassertemperatur, den Wasserdruck, den Wasserdurchfluss und/oder für die Trinkwassergüte des im Trinkwasserversorgungssystem 2 geführten Wassers.

Figur 1a zeigt exemplarisch einen ersten Sensor 24 im Warmwasserstrang 14 des Unterversorgungsstrangs 8 sowie einen Sensor 26 im Kaltwasserstrang 16 des Unterversorgungsstrangs 8. Bei den Sensoren 24, 26 kann es sich beispielsweise um Volumenstromsensoren, die das durch die jeweilige Leitung fließende Wasservolumen pro Zeit messen, um Temperatursensoren, die die Wassertemperatur in der jeweiligen Leitung messen, oder um Drucksensoren handeln, die den Wasserdruck innerhalb der jeweiligen Leitung messen. In den Trinkwassersträngen 14, 16 können auch mehrere dieser Sensoren integriert werden, beispielsweise jeweils ein Volumenstromsensor, ein Temperatursensor und/oder ein Drucksensor. Weiterhin können auch an mehreren Positionen der Trinkwasserstränge 14, 16 entsprechende Sensoren vorgesehen werden, um an verschiedenen Positionen der Trinkwasserstränge 14,16 das durch die Leitungen fließende Wasservolumen, die Wassertemperatur und/oder den Wasserdruck zu messen.

In das Trinkwasserleitungssystem 4 können auch Sensoren integriert sein, die Messwerte für die Trinkwassergüte bestimmen, beispielsweise den pH-Wert, den Härtegrad oder die Konzentration von Schwebestoffen oder Bakterien im Wasser. Beispielsweise kann es sich bei den Sensoren 24, 26 um entsprechende Sensoren handeln. Weiterhin können derartige Sensoren zum Beispiel in der Kaltwasserzuleitung 12 oder der Warmwasserzuleitung 10 des Hauptstrangs 6 oder unmittelbar hinter dem zentralen Einspeisepunkt des örtlichen Wasserversorgers in das Trinkwasserversorgungssystem 2 vorgesehen sein.

Weiterhin sind an den jeweiligen Trinkwasserentnahmestellen Sensoren vorgesehen. So ist zum Beispiel die Duscharmatur 18 für Warm- und Kaltwasser wie in Fig. 1b gezeigt jeweils mit einem Temperatursensor 28 und mit einem Volumenstromsensor 30 ausgestattet, der den Volumenstrom des an der Duscharmatur 18 abgelassene Warm- bzw. Kaltwassers misst. Die Waschtischarmatur 20 weist ebenfalls jeweils einen Temperatursensor 28 und einen Volumenstromsensor 30 auf, der den Volumenstrom des an der Waschtischarmatur 20 abgelassenen Wassers misst. Schließlich weist auch die WC-Spülung 22 einen Temperatursensor 28 sowie einen Volumenstromsensor 30 für das an der WC-Spülung abgelassene Kaltwasser auf.

Neben den einzelnen Sensoren weist das Trinkwasserversorgungssystem 2 eine zentrale Steuereinrichtung 40 auf, die die von den Sensoren erfassten Messwerte empfangen und auswerten kann. Zur Übermittlung der Messwerte von den Sensoren an die zentrale Steuereinrichtung 40 ist bei dem in Fig. 1a-b gezeigten Ausführungsbeispiel ein Feldbus 42 vorgesehen, an den die einzelnen Sensoren und die zentrale Steuereinrichtung 40 angeschlossen sind. Alternativ dazu kann auch eine sternförmige Verbindung der Sensoren mit der zentralen Steuereinrichtung 40 vorgesehen sein. Weiterhin sind auch drahtlose Kommunikationsverbindungen zwischen einzelnen Sensoren und der zentralen Steuereinrichtung denkbar, beispielsweise über Funk, WLAN, Bluetooth oder dergleichen.

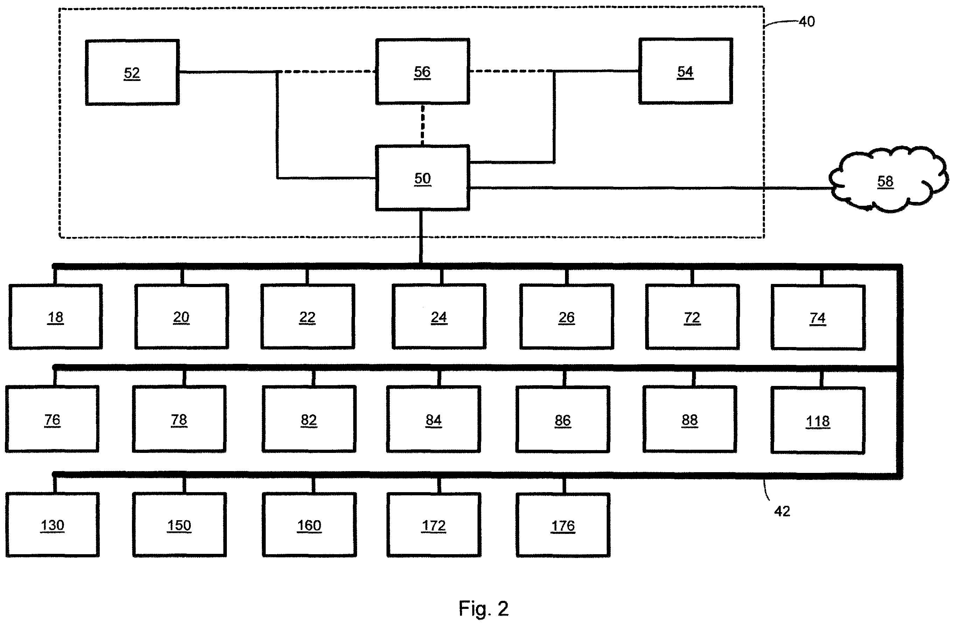

Figur 2 zeigt einen möglichen Aufbau der zentralen Steuereinrichtung 40 aus Figur 1a. Die zentrale Steuereinrichtung 40 umfasst einen Controller 50, der über den Feldbus 42 die Messwerte der an den Feldbus angeschlossenen Sensoren empfangen kann. Bei dem Controller kann es sich beispielsweise um eine elektronische Schaltung mit mindestens einem programmierbaren Mikrokontroller handeln.

Weiterhin umfasst die zentrale Steuereinrichtung 40 eine oder mehrere Nutzerschnittstellen 52, auf denen vom Controller 50 empfangene und/oder ausgewertete Daten angezeigt werden können. Beispielsweise kann der Controller 50 über die Nutzerschnittstelle 52 die Messwerte der Temperatursensoren in einem Unterversorgungsstrang 8 anzeigen, sodass ein Nutzer an der Nutzerschnittstelle 52 sofort einen Überblick über die Wassertemperaturen in dem gesamten Unterversorgungsstrang erhält.

Neben der Nutzerschnittstelle 52 ist eine elektronische Schnittstelle 54 vorgesehen, über die die von dem Controller 50 empfangenen beziehungsweise ausgewerteten Daten zur weiteren Verarbeitung oder Speicherung an einen externen Computer übergeben werden können. Auf diese Weise kann beispielsweise eine weitere Auswertung oder Archivierung der Messdaten mit Hilfe des externen Computers erfolgen.

Die zentrale Steuereinrichtung 40 kann darüber hinaus auch noch ein Frontend 56 aufweisen, das mit den vom Controller 50 empfangenen und/oder ausgewerteten Daten versorgt wird. Im Frontend 56 können dann weitere Auswertungen erfolgen oder nutzergesteuerte Auswertungen durchgeführt werden. Es kann auch die gesamte Auswertung auf das Frontend 56 verlagert werden, so dass die von den Sensoren empfangenen Messdaten durch den Controller 50 nur an das Frontend 56 weitergeleitet werden müssen.

Bei dem Frontend 56 kann es sich beispielsweise um das Frontend einer bestehenden Gebäudeautomatisation, beispielsweise von einer Gebäudelüftungs- oder einer Heizungsanlage, handeln. Auf diese Weise können mehrere Gewerke eines Gebäudes oder einer Einrichtung von einer zentralen Stelle überwacht und/oder gesteuert werden. Vorzugsweise weist das Frontend 56 mindestens einen Mikroprozessor und einen Speicher auf, auf dem ein Computerprogramm mit Befehlen zur Darstellung und/oder Auswertung der von den Sensoren übermittelten Messdaten gespeichert ist.

Das Frontend 56 kann bei Bedarf auch Ausgaben über die Nutzerschnittstelle 52 oder über die Schnittstelle 54 bewirken, insbesondere wenn die Auswertung der Messdaten auf dem Frontend 56 erfolgt. Der Controller 50 oder auch das Frontend 56 können beispielsweise auch mit einem Computernetzwerk oder einer Cloud 58 verbunden sein, zum Beispiel um Messdaten oder daraus berechnete Größen zu speichern oder Steuerbefehle abzurufen.

Durch das Vorsehen der zentralen Steuereinrichtung 40 ist eine zentrale Auswertung der von den einzelnen Sensoren gemessenen Messwerte möglich, sodass der Zustand des Trinkwasserversorgungssystems 2 an einer zentralen Stelle ausgewertet und gegebenenfalls beurteilt werden kann.

Weiterhin kann vorgesehen sein, dass das Trinkwasserversorgungssystems 2 von der zentralen Steuereinrichtung 40 aus gesteuert werden kann.

Zu diesem Zweck umfasst das Trinkwasserversorgungssystem 2 mehrere dezentrale Steuerelemente, mit denen der Wasserdurchfluss und die Wassertemperatur an verschiedenen Stellen im Trinkwasserleitungssystem 4 beeinflusst werden kann.

In den Figuren 1a-b sind exemplarisch folgende Steuerelemente gezeigt, die im Folgenden erläutert werden:
- ein Steuerelement 70 an der WC-Spülung 22,
- eine jeweilige ansteuerbare gesonderte Spüleinheit 72, 74 an dem Warm- und Kaltwasserstrang 14,16,
- eine jeweilige ansteuerbare Pumpe 76, 78 in der Warmwasserzuleitung 10 und der Kaltwasserzuleitung 12 des Hauptversorgungsstrangs 6,
- ein jeweils ansteuerbares Drosselventil 82, 84 am jeweiligen Ende des Warm- und Kaltwasserstrangs 14, 16 und
- eine ansteuerbare Kühlstrecke 86.

Durch das Vorsehen der jeweiligen Steuerelemente und der zentralen Steuereinrichtung 40 zur Ansteuerung dieser Steuerelemente ist es möglich, das Trinkwasserversorgungssystem 2 von zentraler Stelle zu steuern und gegebenenfalls zu regeln. Beispielsweise kann ein Nutzer durch Eingabe eines entsprechendes Befehls über die Nutzerschnittstelle 52 oder das Frontend 56 ein oder mehrere der dezentralen Steuerelemente von zentraler Stelle aus ansteuern.

Im Folgenden wird die Funktion der einzelnen Steuerelemente erläutert:
Mit dem Steuerelement 70 an der WC-Spülung 22 kann ein Spülvorgang initiiert werden, so dass Wasser aus dem Kaltwasserstrang 16 aus dem Trinkwasserleitungssystem 4 abgelassen wird. Das Steuerelement 70 und die WC-Spülung stellen damit eine ansteuerbare Spüleinheit dar.

Stellt ein Nutzer beispielsweise anhand der über die Nutzerschnittstelle 52 ausgegebenen Informationen fest, dass der Bereich des Kaltwasserstrangs 16, in dem sich die WC-Spülung 22 befindet, über einen langen Zeitraum nicht gespült wurde und das Wasser für eine lange Zeit im Kaltwasserstrang 16 stand, so kann er über die zentrale Steuereinrichtung 40 und das dadurch angesteuerte Steuerelement 70 eine Spülung initiieren. Dadurch wird ein Spülvorgang durchgeführt und Wasser aus dem entsprechenden Leitungsabschnitt des Kaltwasserstrangs 16 abgelassen, so dass frisches Wasser in den entsprechenden Abschnitt des Kaltwasserstrangs 16 nachströmen kann. Ähnliche Steuerelemente 70 können auch an anderen Trinkwasserentnahmestellen vorgesehen sein, beispielsweise an der Duscharmatur 18 oder der Waschtischarmatur 20. Insbesondere kann über die Duscharmatur 18 oder die Waschtischarmatur 20 auch eine Spülung des Warmwasserstrangs 14 durchgeführt werden.

Der Nutzer kann einen solchen Spülvorgang beispielsweise auch dann initiieren, wenn er über die an der Nutzerschnittstelle 52 angezeigten Informationen feststellt, dass die Wassertemperatur in einem bestimmten Leitungsabschnitt des Kaltwasserstrangs 16 zu hoch ist oder in einem bestimmten Leitungsabschnitt des Warmwasserstrangs 14 zu niedrig ist.

In gleicher Weise kann ein Spülvorgang des Warm- oder Kaltwasserstrangs auch an einer jeweiligen gesonderten Spüleinheit 72, 74 durchgeführt werden. Mit einer solchen Spüleinheit kann aus dem jeweiligen Strang unabhängig von den Trinkwasserentnahmestellen Wasser abgelassen werden. Eine solche Spüleinheit kann beispielsweise einen in den jeweilige Strang integrierten Leitungsauslass mit einem ansteuerbaren Ventil aufweisen, so dass durch Öffnen des Ventils Wasser durch den Leitungsauslass aus dem Strang abgelassen und zum Beispiel in einen darunter vorgesehenen Abfluss geleitet werden kann.

Durch einen zentral initiierten Spülvorgang an einer Trinkwasserentnahmestelle oder einer Spüleinheit kann auch die in dem jeweiligen Trinkwasserstrang geführte Wassermenge bzw. der Wasserdruck innerhalb des jeweiligen Trinkwasserstrangs beeinflusst werden.

Das Trinkwasserversorgungssystem 2 weist weiterhin Präsenzmelder 88 in Form von Bewegungsmeldern auf. Über die Präsenzmelder 88 erhält die zentrale Steuereinrichtung 40 eine Information darüber, ob sich eine Person im Bereich einer der Trinkwasserentnahmestellen 18, 20 aufhält. Vorzugsweise ist die zentrale Steuereinrichtung dazu eingerichtet, die Durchführung eines zentral initiierten Spülvorgangs an einer der Trinkwasserentnahmestellen 18, 20 zu unterbrechen bzw. zu unterbinden, wenn der entsprechende Präsenzmelder 88 die Anwesenheit einer Person detektiert. Auf diese Weise kann verhindert werden, dass eine Person im Bereich der Trinkwasserentnahmestellen 18, 20 durch einen automatisch initiierten Spülvorgang nass gespritzt oder - bei einer Heißwasserspülung - verbrüht wird.

Weiterhin kann ein Präsenzmelder wie der Präsenzmelder 88 auch dazu verwendet werden, um die Steuerung des Trinkwasserversorgungssystems 2 durch die zentrale Steuereinrichtung 40 automatisch zwischen einem Normalmodus und einem Abwesenheitsmodus, beispielsweise einem Urlaubsmodus, umzuschalten. Zu diesem Zweck kann die Steuereinrichtung 40 dazu eingerichtet sein, automatisch von einem Normalmodus in einen Abwesenheitsmodus umzuschalten, wenn über einen vorgegebenen Zeitraum mit dem Präsenzmelder 88 oder mit weiteren vorgesehenen Präsenzmeldern keine Person detektiert wurde. Weiterhin kann die Steuereinrichtung 40 dazu eingerichtet sein, automatisch in einen Normalmodus zurückzuschalten, wenn während des Abwesenheitsmodus von einem Präsenzmelder eine Person detektiert wird. Für den Normalmodus und den Abwesenheitsmodus können in der zentralen Steuereinrichtung 40 zum Beispiel verschiedene Steuerprogramme hinterlegt sein, die verschiedene Befehle zur Steuerung des Trinkwasserversorgungssystems 2 im Normal- bzw. Abwesenheitsmodus enthalten.

Über die Pumpen 76, 78 kann der Wasserdruck innerhalb der Warm- und/oder Kaltwasserzuleitung 10,12 bzw. die durch die Warm- und/oder Kaltwasserzuleitung 10, 12 fließende Wassermenge beeinflusst werden. Stellt der Nutzer beispielsweise anhand der Nutzerschnittstelle 52 fest, dass die für die einzelnen Trinkwasserentnahmestellen zur Verfügung stehende Wassermenge oder der Wasserdruck zu gering ist, so kann er über die zentrale Steuereinrichtung 40 eine Leistungserhöhung der Pumpen 76, 78 bewirken.

Zusätzlich oder alternativ zu den Pumpen 76, 78 können auch in Unterversorgungssträngen, beispielsweise in dem Unterversorgungsstrang 8, Pumpen vorgesehen sein, um lokal den Wasserdurchfluss bzw. den Druck zu steuern.

Der Warmwasserstrang 14 und der Kaltwasserstrang 16 sind jeweils über ein Drosselventil 82, 84 an eine jeweilige Zirkulationsleitung 90, 92 angeschlossen, über die Wasser innerhalb des Trinkwasserleitungssystems 4 zirkuliert werden kann. Auf diese Weise kann das Wasser aus dem Warmwasserstrang 14 bzw. dem Kaltwasserstrang 16 abgeführt werden, ohne Wasser aus dem Trinkwasserversorgungssystem 2 auslassen zu müssen. Die Zirkulationsleitungen 90, 92 sind im vorliegenden Ausführungsbeispiel an eine entsprechende zentrale Warmwasser-Zirkulationsleitung 94 ("ZW" in Fig. 1a) und Kaltwasser-Zirkulationsleitung 96 ("KW" in Fig. 1a) im Hauptversorgungsstrang 6 angeschlossen, über die das Wasser innerhalb des Trinkwasserleitungssystems 4 wieder zur Entnahme zur Verfügung gestellt werden kann. Beispielsweise kann die Warmwasser-Zirkulationsleitung 94 das Wasser zu einer Therme leiten, in der es aufgeheizt wird, bevor es dann wieder in die Warmwasserzuleitung 10 eingespeist wird. Die Kaltwasser-Zirkulationsleitung 96 kann das Wasser beispielsweise zu der ansteuerbaren Kühlstrecke 86 leiten, in der das Wasser gekühlt wird, bevor es dann wieder in die Kaltwasserzuleitung 12 eingespeist wird.

Stellt ein Nutzer beispielsweise über die Nutzerschnittstelle 52 fest, dass das Wasser in dem Warmwasserstrang 14 oder dem Kaltwasserstrang 16 zu lange steht oder außerhalb des gewünschten Temperaturbereichs liegt, so kann er durch Ansteuerung des entsprechenden Drosselventils 82 oder 84 Wasser aus dem Warmwasserstrang 14 oder dem Kaltwasserstrang 16 über die entsprechende Zirkulationsleitung 90, 92 abführen, so dass frisches Wasser nachströmt.

Da die Zirkulationsleitungen 90, 92 es erlauben, Wasser aus dem Warm- bzw. Kaltwasserstrang 14, 16 abzuführen, ohne es aus dem Trinkwasserversorgungssystem 2 auslassen zu müssen, kann ein Wasseraustausch im Trinkwasserversorgungssystem erfolgen, ohne dass Wasser unnötig verschwendet wird. Insbesondere kann das durch die Zirkulationsleitungen 90, 92 abgeführte Wasser im Trinkwasserversorgungssystem 2 weiterverwendet werden.

Das Vorsehen einer Zirkulationsleitung ist besonders in einem Kaltwasserstrang vorteilhaft, da das Wasser auf diese Weise abgeführt werden kann, wenn es sich durch zu langes Stehen in dem Kaltwasserstrang über eine vorgegebene Maximaltemperatur erwärmt hat. Durch die ansteuerbare Kühlstrecke 86 kann das Wasser in diesem Fall wieder auf die gewünschte Temperatur heruntergekühlt werden.

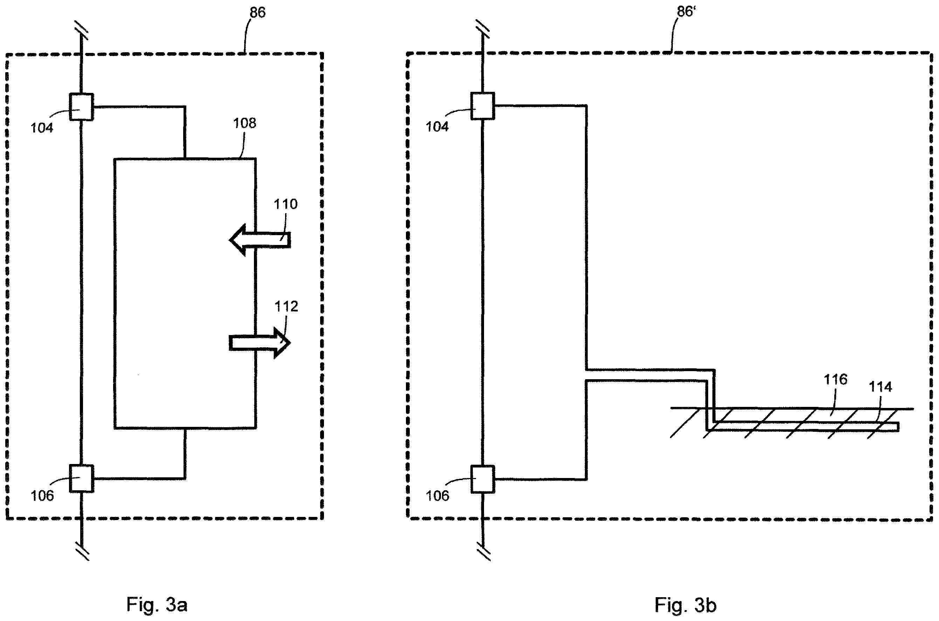

Figur 3a zeigt einen möglichen Aufbau der Kühlstrecke 86. Die Kühlstrecke 86 ist über zwei ansteuerbare Abzweigventile 104 und 106 an die Kaltwasser-Zirkulationsleitung 94 angeschlossen. Durch Ansteuerung der Abzweigventile 104 und 106 kann das durch die Kaltwasser-Zirkulationsleitung 94 fließende Wasser in die Kühlstrecke 86 umgeleitet werden. In der Kühlstrecke 86 ist ein Wärmetauscher 108 mit einer Kühlmittelzuführung 110 und eine Kühlmittelabführung 112 angeordnet, durch den das durch den Wärmetauscher 108 fließende Wasser gekühlt werden kann, um die gewünschte Wassertemperatur für die Kaltwasserzuleitung 12 zu erreichen.

Figur 3b zeigt eine alternative Kühlstrecke 86'. Die Kühlstrecke 86' unterscheidet sich dadurch von der Kühlstrecke 86, dass anstelle einer aktiven Kühlung über einen mit einem Kühlmittel betriebenen Wärmetauschers 108 eine passive Kühlung erfolgt, indem die Kühlstrecke 86' einen Leitungsabschnitt 114 umfasst, der durch eine kalte Umgebung wie zum Beispiel einen Kellerbereich oder, wie in Fig. 3b angedeutet, das Erdreich 116 geführt wird.

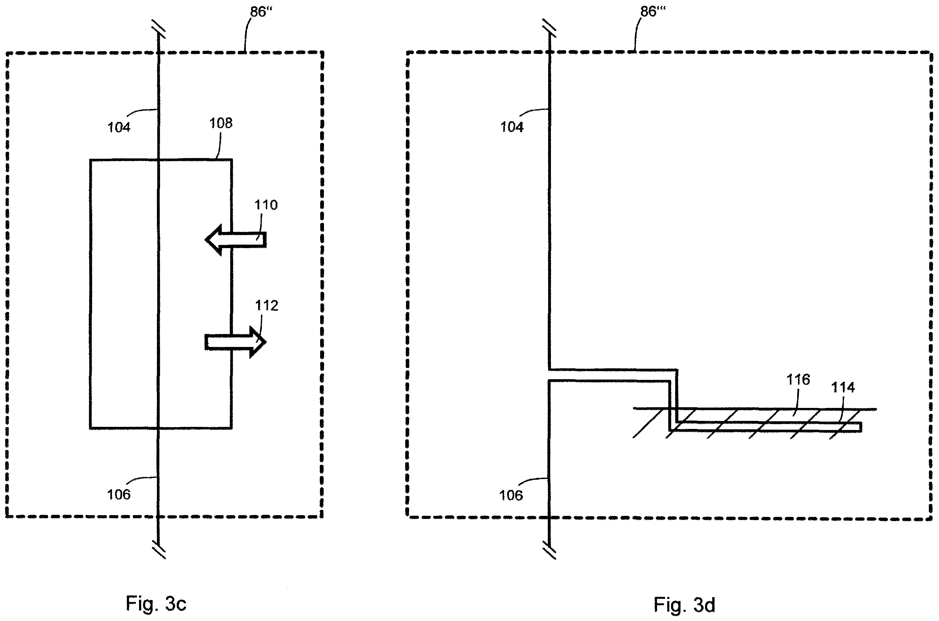

Figur 3c zeigt eine weitere alternative Kühlstrecke. Die Kühlstrecke 86" weist wie die Kühlstrecke 86 einen Wärmetauscher 108 mit einer Kühlmittelzuführung 110 und einer Kühlmittelabführung 112 auf. Anders als bei der Kühlstrecke 86 ist der Wärmetauscher 108 jedoch direkt an die Kaltwasser-Zirkulationsleitung 94 angeschlossen. Durch Aktivieren bzw. Deaktivieren der Kühlmittelzuführung 110 kann das durch den Wärmetauscher 108 fließende Wasser zeit- bzw. bedarfsweisegekühlt werden, um die gewünschte Wassertemperatur für die Kaltwasserzuleitung 12 zu erreichen. Alternativ ist auch eine permanente Kühlung möglich.

Wie der Wärmetauscher 108 kann auch der Leitungsbereich 114 der Kühlstrecke 86' direkt an die Kaltwasser-Zirkulationsleitung 94 angeschlossen werden; dies wird bei der Kühlstrecke 86‴ in Figur 3d illustriert.

Das direkte Anschließen des Wärmetauschers 108 bzw. des Leitungsbereichs 114 an die Kaltwasser-Zirkulationsleitung 94 hat den Vorteil, dass Totwasser vermieden wird, wie es bei den Kühlstrecken 86 und 86' im jeweils ungenutzten Leitungsabschnitt zwischen den beiden Abzweigventilen 104 und 106 anfallen kann.

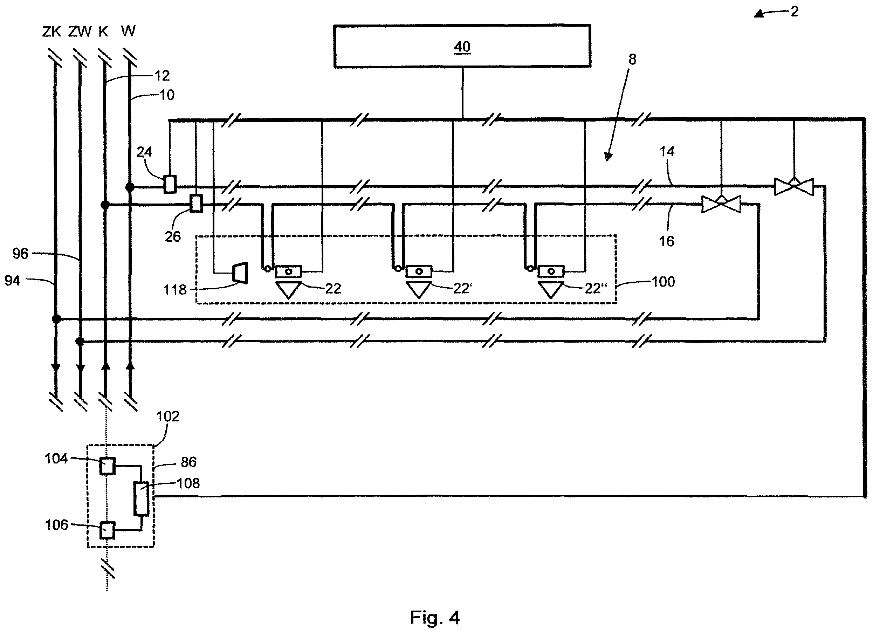

Figur 4 zeigt einen weiteren Ausschnitt des Trinkwasserversorgungssystems 2 aus Figur 1a. Der Übersicht halber wurden in Figur 4 einige Komponenten aus Figur 1a weggelassen und andere Komponenten, die in Figur 1a nicht dargestellt sind, abgebildet. Wie Figur 4 zeigt, können an den Unterversorgungsstrang 8 nicht nur die in Figur 1a gezeigten Trinkwasserentnahmestellen 18, 20 und 22 sondern weitere Trinkwasserentnahmestellen angeschlossen sein, beispielsweise alle Trinkwasserentnahmestellen einer Station eines Krankenhauses. In Figur 4 sind exemplarisch neben der WC-Spülung 22 weitere WC-Spülungen 22' und 22" dargestellt. Alle WC-Spülungen 22, 22' und 22" sind analog zur WC-Spülung 22 mit jeweiligen Temperatursensoren 28, Volumenstromsensoren 30 und Steuerelementen 70 zur Initiierung einer Spülung versehen.

Die Steuereinrichtung 40 ermöglicht eine gruppenweise Ansteuerung der in das Trinkwasserversorgungssystem 2 integrierten Steuerelemente. So sind in Figur 4 beispielsweise alle WC-Spülungen 22, 22' und 22" des Unterversorgungsstrangs 8 zu einer virtuellen Gruppe 100 zusammengefasst und die Steuereinrichtung 40 ist dazu eingerichtet, die Steuerelemente 70 der jeweiligen WC-Spülungen gemeinsam anzusteuern. Beispielsweise kann der Controller 50 dazu eingerichtet sein, über die Nutzerschnittstelle 52 einen Befehl zur Spülung sämtlicher WC-Spülungen im Unterversorgungsstrang 8 zu empfangen und als Reaktion darauf die einzelnen Steuerelemente 70 der WC-Spülungen aus der Gruppe 100 derart anzusteuern, dass ein Spülvorgang an allen WC-Spülungen der Gruppe 100 durchgeführt wird. Hierdurch wird ein großer, vorzugsweise turbulenter Volumenstrom im Leitungssystem, insbesondere im Unterversorgungsstrang 8, erreicht. Durch einen turbulenten Volumenstrom können insbesondere die Rohrwände von Verunreinigungen wie zum Beispiel einem Biofilm befreit werden.

Auch Steuerelemente der Kühlstrecke 86 können zu einer Gruppe zusammengefasst sein. Beispielsweise können die beiden ansteuerbaren Abzweigventile 104 und 106 zu einer virtuellen Gruppe zusammengefasst werden, so dass diese durch einen einzelnen Befehl in eine Position geschaltet werden, in der das Wasser durch die Kühlstrecke 86 geleitet wird, oder alternativ in eine Position geschaltet werden, in der das Wasser an der Kühlstrecke 86 vorbeigeleitet wird. Weiterhin kann auch der Wärmetauscher 108 in die virtuelle Gruppe integriert werden, so dass mit dem Aktivieren der Kühlstrecke 86 über die Abzweigventile 104 und 106 zum Beispiel ein Kompressor bzw. eine Pumpe für das Kühlmedium gestartet werden.

Die zentrale Steuereinrichtung 40 kann weiterhin dazu eingerichtet sein, mittels entsprechender Sensoren an den WC-Spülungen 22, 22`, 22" im Unterversorgungsstrang 8 zu bestimmen, ob der Unterversorgungsstrang 8 durch eine Spülung an einer der WC-Spülungen 22, 22', 22" innerhalb eines vorgegebenen Zeitraums zumindest einmalig gespült wurde und, wenn dies nicht der Fall ist, an einzelnen der WC-Spülungen 22, 22', 22" eine entsprechende Spülung automatisch herbeizuführen. Eine solche zentrale Überwachung der Spülungen im Unterversorgungsstrang 8 spart gegenüber einer autarken und einzelnen Überwachung an jeder einzelnen WC-Spülung Wasser, da seltener und mit weniger Wasser gespült werden muss.

Ein von der zentralen Steuereinrichtung 40 initiierter Spülvorgang, insbesondere an mehreren WC-Spülungen 22, 22', 22" gleichzeitig, kann zu einer erheblichen Lärmbelästigung führen. Aus diesem Grund ist die zentrale Steuereinrichtung 40 vorzugsweise dazu eingerichtet, die automatischen Spülvorgänge abhängig von der Tageszeit durchzuführen. Zu diesem Zweck kann der Controller 50 beispielsweise eine Systemuhr aufweisen oder mit einer solchen verbunden sein, die eine Information über die aktuelle Tageszeit zur Verfügung stellt. Auf diese Weise können beispielsweise auf einer Krankenhausstation automatische Spülungen während der Nachtruhe unterdrückt werden. In einem Bürogebäude können Spülungen auch gezielt nachts durchgeführt werden, wenn in dem Bürogebäude nicht gearbeitet wird.

Um eine Lärmbelästigung durch automatische Spülvorgänge weiter zu reduzieren, weist das Trinkwasserversorgungsystem 2 weiterhin einen Akustiksensor 118 in Form eines Mikrofons auf, der der zentralen Steuereinrichtung 40 einen Messwert über die Lautstärke in einem zu überwachenden Bereich, beispielsweise auf einer Krankenhausstation, zur Verfügung stellt. Die zentrale Steuereinrichtung 40 ist vorzugsweise dazu eingerichtet, Spülvorgänge nur dann automatisch zu initiieren, wenn die vom Akustiksensor 118 bestimmte Lautstärke unterhalb einer vorgegebenen Maximallautstärke liegt. Weiterhin ist die zentrale Steuereinrichtung 40 dazu eingerichtet, einen automatisch initiierten Spülvorgang abzubrechen, wenn dadurch die Lautstärke über einen vorgegebene Maximallautstärke ansteigt. Auf diese Weise wird ein Komfortgewinn erzielt.

Bei einem weiteren Ausführungsbeispiel können die Sensoren 24, 26 dazu eingerichtet sein, Messwerte für die Geschwindigkeitsverteilung des Wassers im Warm- bzw. Kaltwasserstrang zu ermitteln. Auf diese Weise lässt sich feststellen, ob das Wasser turbulent oder laminar fließt. Wird beispielsweise bei einer Spülung des Kaltwasserstrangs durch eine automatisch initiiert Spülung an mehreren der Trinkwasserentnahmestellen 22, 22', 22" festgestellt, dass die Wasserströmung im Kaltwasserstrang 16 laminer ist, so kann die Steuereinrichtung 40 dazu eingerichtet sein, weitere Spülungen zu initiieren, um höhere Fließgeschwindigkeiten und dadurch eine turbulente Strömung zu erreichen, da durch eine turbulente Strömung eine zuverlässigere Spülung des Kaltwasserstrangs 16 bewirkt werden kann als durch eine laminare Strömung, insbesondere was die Reinigung der Rohrwandung betrifft. Bei einer laminaren Strömung geht die Strömungsgeschwindigkeit an der Rohrwandung gegen Null, während bei einer turbulenten Strömung dort durch Wirbel hohe Strömungsgeschwindigkeiten auftreten.

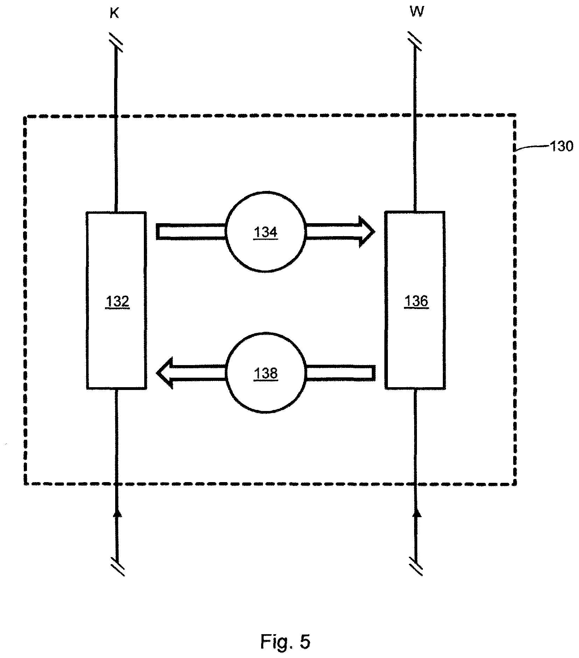

Fig. 5 zeigt eine Wärmepumpe 130, die zwischen der Warmwasserzuleitung 10 und der Kaltwasserzuleitung 12 des Hauptversorgungsstrangs 6 des Trinkwasserversorgungssystems 2 vorgesehen ist. Die Wärmepumpe 130 umfasst einen mit der Kaltwasserzuleitung 12 gekoppelten Verdampfer 132, in dem ein Wärmetransportmedium verdampft, einen Verdichter 134 zum Verdichten des verdampften Wärmetransportmediums, einen mit der Warmwasserzuleitung 10 gekoppelten Verflüssiger 136 zum Verflüssigen des verdichteten Wärmetransportmediums und ein Entspannungsventil 138 zum Entspannen des verflüssigen Wärmetransportmediums. Durch die zum Betrieb des Verdichters 134 aufgewendete Energie wird mit der Wärmepumpe 130 ein Wärmefluss von der Kaltwasserzuleitung 12 zur Warmwasserzuleitung 10 erreicht, so dass das Wasser in der Kaltwasserzuleitung 12 gekühlt und das Wasser in der Warmwasserzuleitung 10 erhitzt wird. Auf diese Weise lassen sich auf ressourcenschonende Weise eine gleichzeitige Kühlung des Kaltwassers und ein Erhitzen des Warmwassers erreichen.

Eine der Wärmepumpe 130 entsprechende Wärmepumpe kann beispielsweise auch zwischen dem Warm- und Kaltwasserstrang 14, 16 des Unterversorgungsstrangs 8 vorgesehen sein.

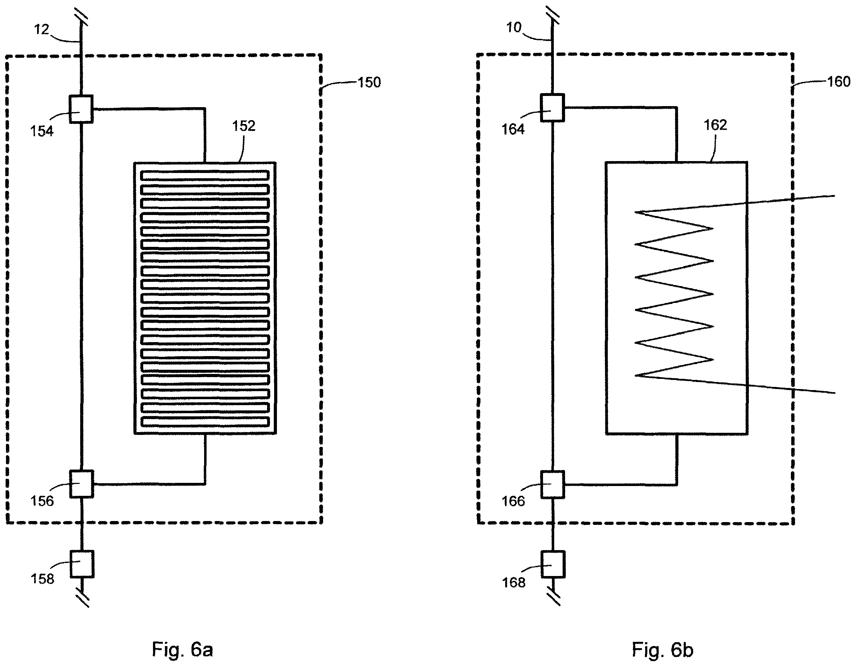

Fig. 6a-b zeigen zwei Ausführungsbeispiele für weitere Steuerelemente des Trinkwasserversorgungssystems 2 aus Fig. 1a. Fig. 6a zeigt ein ansteuerbares Filterelement 150 und Fig. 6b ein Sterilisationselement 160. Das Filterelement 150 bzw. das Sterilisationselement 160 können zum Beispiel in die Kaltwasserzuleitung 12 und/oder in die Warmwasserzuleitung 10 integriert sein. Ebenso ist es möglich, ein entsprechendes Filterelement bzw. Sterilisationselement unmittelbar hinter den zentralen Einspeisepunkt des örtlichen Wasserversorgers in das Trinkwasserversorgungssystem 2 zu integrieren.

Das Filterelement 150 in Fig. 6a umfasst einen Filter 152, zum Beispiel einen Plattenfilter, sowie zwei ansteuerbare Abzweigventile 154, 156, mit denen das Wasser aus der Kaltwasserzuleitung 12 durch den Filter 152 geleitet werden kann. Durch den Filter 152 können beispielsweise Schwebestoffe oder Bakterien aus dem Wasser gefiltert werden.

Zur geregelten Ansteuerung des Filterelements 150 kann das Trinkwasserversorgungssystem 2 einen Sensor 158 aufweisen, der die Konzentration von Schwebestoffen oder Bakterien in dem durch das Filterelement 150 geleiteten Wasser misst. Die zentrale Steuereinrichtung 40 kann dann beispielsweise dazu eingerichtet sein, eine automatische Ansteuerung der Abzweigventile 154, 156 vorzunehmen, wenn die gemessene Schwebestoff- bzw. Bakterienkonzentration eine vorgegebene Maximalkonzentration überschreitet, so dass das Wasser durch den Filter 152 geleitet wird.

Das Sterilisationselement 160 in Fig. 6b umfasst eine Sterilisationsstrecke 162 sowie zwei ansteuerbare Ventile 164, 166, mit denen das Wasser aus der Warmwasserzuleitung 10 durch die Sterilisationsstrecke 162 geleitet werden kann. In der Sterilisationsstrecke 162 wird das Wasser sterilisiert, beispielsweise durch Einwirkung von Wärme (wie in Fig. 6b illustriert) oder auch durch Beleuchtung mit intensivem UV-Licht.

Zur geregelten Ansteuerung des Sterilisationselements 160 kann das Trinkwasserversorgungssystem einen Sensor 168 aufweisen, der die Konzentration von Bakterien in dem durch das Sterilisationselement 160 geleiteten Wasser misst. Die zentrale Steuereinrichtung 40 kann dann beispielsweise dazu eingerichtet sein, eine automatische Ansteuerung der Ventile 164, 166 vorzunehmen, wenn die gemessene Bakterienkonzentration eine vorgegebene Maximalkonzentration überschreitet, so dass das Wasser durch die Sterilisationsstrecke 162 geleitet wird.

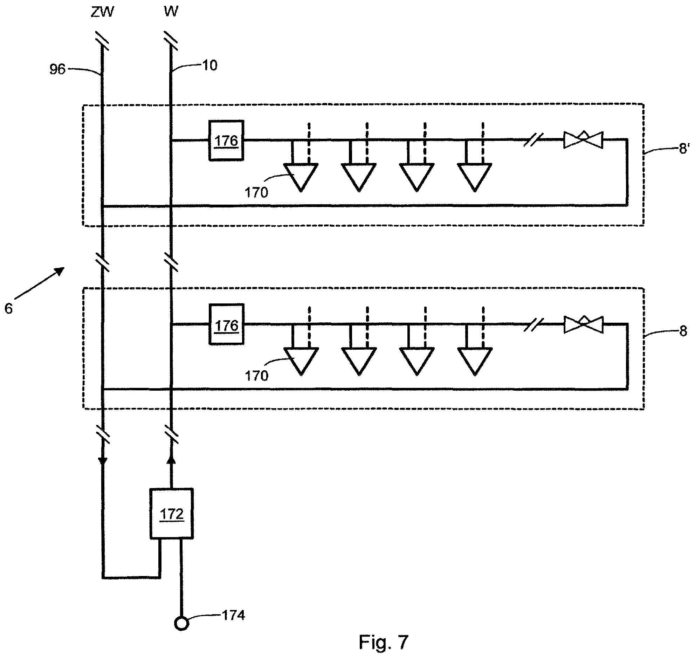

Figur 7 zeigt einen weiteren Ausschnitt des Trinkwasserversorgungssystems 2 aus Fig. 1a. Der Übersicht halber wurden in Figur 7 einige Komponenten aus Figur 1a bzw. 4 weggelassen und andere Komponenten, die in Figur 1a bzw. 4 nicht dargestellt sind, abgebildet. Figur 7 zeigt die Warmwasserzuleitung 10 und die Warmwasser-Zirkulationsleitung 96 des Hauptversorgungsstrangs 6. Die Kaltwasserzuleitung 12 und die Kaltwasser-Zirkulationsleitung 94 sind in Fig. 7 der Übersicht halber weggelassen.

Am Hauptversorgungsstrang 6 sind mehrere Unterversorgungsstränge 8, 8' angeschlossen, die vom Hauptversorgungsstrang 6 gespeist werden und zum Beispiel verschiedene Etagen eines größeren Gebäudekomplexes wie zum Beispiel eines Krankenhauses versorgen. In die Unterversorgungsstränge 8, 8' sind eine Mehrzahl verschiedener Trinkwasserentnahmestellen 170 integriert, von denen in Fig. 7 einige dargestellt sind.

Im Trinkwasserversorgungssystem 2 ist eine zentrale Warmwassertherme 172 vorgesehen, mit der unter anderem von einem zentralen Einspeisepunkt 174 des örtlichen Wasserversorgers zur Verfügung gestelltes Wasser auf die gewünschte Wassertemperatur für die Warmwasserversorgung erhitzt werden kann. Weiterhin kann auch die Warmwasser-Zirkulationsleitung 96 das im Leitungssystem zirkulierte Wasser zur Warmwassertherme 172 zurückführen, um es dort erneut zu erhitzen.

Die zentrale Warmwassertherme 172 ist dazu ausgelegt, Wasser von Raumtemperatur auf die gewünschte Temperatur von beispielsweise 65 °C zu erhitzen. Weiterhin ist der Durchsatz der zentralen Warmwassertherme 172 dazu ausgelegt, um den gesamten Warmwasserteil des Trinkwasserversorgungssystems 2 und insbesondere sämtliche darin integrierten Trinkwasserentnahmestellen 170 mit Warmwasser zu versorgen.

Bei größeren Gebäudekomplexen wie zum Beispiel einem Krankenhaus können zum Teil große Leitungsstrecken zwischen der zentralen Warmwassertherme 172 und den einzelnen Unterversorgungssträngen 8, 8' liegen. Trotz Leitungsisolierung kann das Wasser dann bereits soweit abgekühlt sein, dass es bereits nach recht kurzer Zeit abgelassen oder über die Warmwasser-Zirkulationsleitung 96 zurück zur zentralen Warmwassertherme 172 transportiert werden müsste.

Um einen ökonomischeren Betrieb des Trinkwasserleitungssystems 2 zu ermöglichen, sind in einzelne Unterversorgungsstränge 8, 8' dezentrale Warmwasserthermen 176 integriert, mit denen das Wasser im Warmwasserstrang des jeweiligen Unterversorgungsstrangs 8, 8' wieder auf die gewünschte Temperatur erhitzt werden kann, ohne dass es über die lange Warmwasser-Zirkulationsleitung 96 zurück zur zentralen Warmwassertherme 172 transportiert werden muss.

Da das Wasser bereits durch die zentrale Warmwassertherme 172 vorgeheizt ist, müssen die dezentralen Warmwasserthermen 176 nur auf eine geringere Temperaturdifferenz ausgelegt sein, beispielsweise um Wasser von 50 °C auf 65 °C zu erhitzen. Weiterhin muss der Durchsatz der dezentralen Warmwasserthermen 176 nur an den Durchsatz des jeweiligen Unterversorgungsstrangs 8, 8' angepasst werden. Auf diese Weise können für die dezentralen Warmwasserthermen 176 kompakt dimensionierte Geräte verwendet werden. Zudem wird eine höhere Modularisierbarkeit und Skalierbarkeit von Gebäuden erreicht. Beispielsweise können einzelne dezentrale Warmwasserthermen in oder außer Betrieb genommen werden, ohne das ganze System zu beeinflussen.

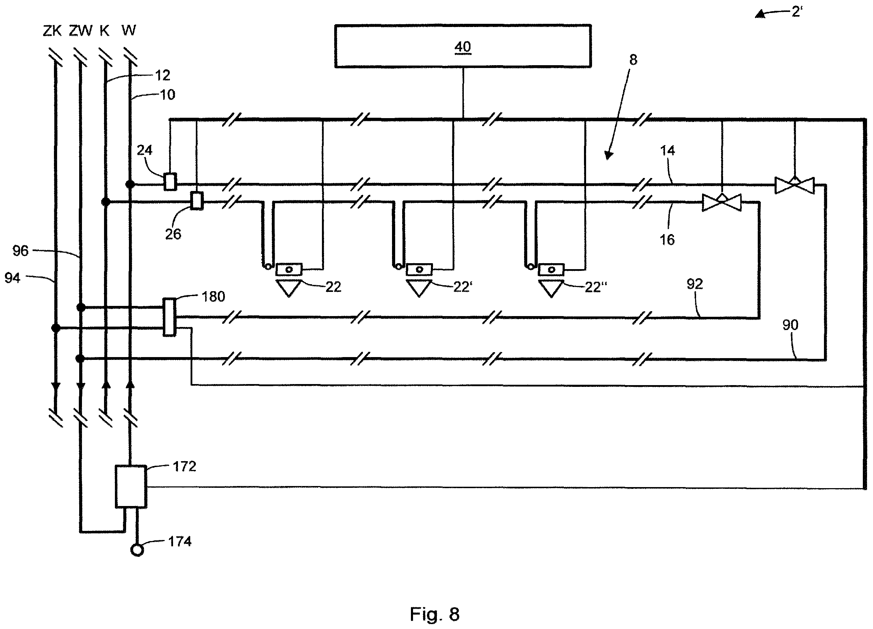

Figur 8 zeigt ein weiteres Ausführungsbeispiel des Systems 2'. Der Aufbau und die Funktionsweise des Systems 2' entsprechen im Wesentlichen dem Aufbau und der Funktionsweise des Systems 2, so dass auf die obige Beschreibung verwiesen wird. Insbesondere sind gleiche Komponenten mit gleichen Bezugszeichen versehen.

Bei dem System 2 ist die Zirkulationsleitung 92 für den Kaltwasserstrang 16 an ein ansteuerbares Dreiwegeventil 180 angeschlossen, so dass das Wasser aus der Zirkulationsleitung 92 wahlweise in die zentrale Kaltwasser-Zirkulationsleitung 94 oder in die zentrale Warmwasser-Zirkulationsleitung 96 geleitet werden kann. Die Steuereinrichtung 40 ist dazu eingerichtet, das Dreiwegeventil 180 derart anzusteuern, dass Wasser aus der Zirkulationsleitung 92 in die zentrale Warmwasser-Zirkulationsleitung 94 geleitet wird, wenn die mit einem Temperatursensor zur Bestimmung der Wassertemperatur im Kaltwasserstrang 16, beispielsweise dem Sensor 26, oder zur Bestimmung der Wassertemperatur in der Zirkulationsleitung 92 einen vorgegebenen Grenzwert überschreitet.

Auf diese Weise kann Wasser, das aufgrund einer Erwärmung im Kaltwasserstrang 16 verkeimt sein könnte, innerhalb des Systems 2' weiterverwendet werden, indem es über die zentrale Warmwasser-Zirkulationsleitung 94 zu der Warmwassertherme 172 geführt wird, in der es erhitzt und dadurch Keime abgetötet werden können.

Anstelle eines Dreiwegeventils 180 kann auch vorgesehen sein, dass das Wasser aus der Zirkulationsleitung 92 grundsätzlich zur zentralen Warmwasser-Zirkulationsleitung 94 geleitet wird.

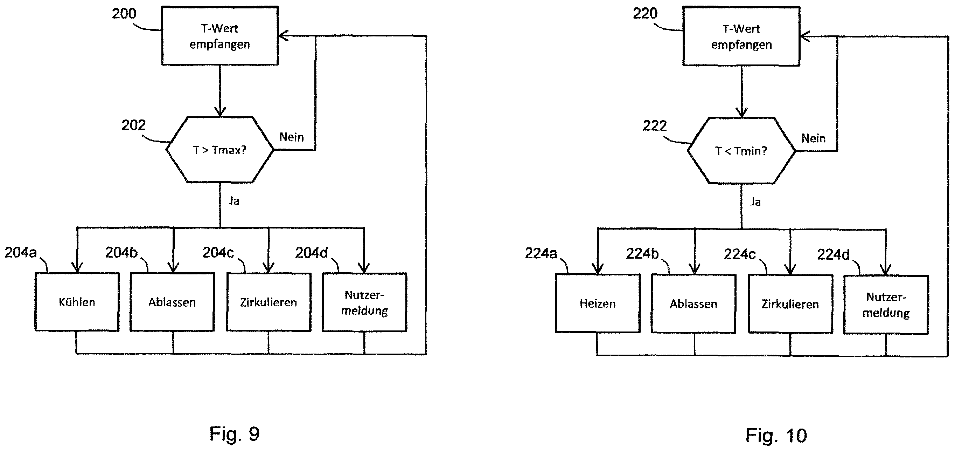

Im Folgenden werden anhand der Figuren 9 bis 14 verschiedene Ausführungsbeispiele des Verfahrens zur Steuerung des Trinkwasserversorgungssystems 2 beschrieben. Insbesondere kann die Steuereinrichtung 40 dazu eingerichtet sein, die Steuerung des Trinkwasserversorgungssystems 2 entsprechend des Verfahrens zu steuern. Zu diesem Zweck kann beispielsweise der Controller 50 einen Speicher aufweisen, auf dem ein Computerprogramm gespeichert ist mit Befehlen, deren Ausführung auf mindestens einem Prozessor des Controllers 50 die Ausführung des jeweiligen Verfahrens bewirkt.

Figur 9 zeigt ein Ausführungsbeispiel des Verfahrens zur Überwachung und Regelung der Kaltwassertemperatur.

Bei dem Verfahren empfängt die zentrale Steuereinrichtung 40 im ersten Schritt 200 Temperaturmesswerte von Temperatursensoren 28, 26 aus dem Kaltwasserstrang, zum Beispiel aus dem Kaltwasserstrang 16 des Unterversorgungsstrangs 8. Im zweiten Schritt 202 wird überprüft, ob die gemessene Temperatur oberhalb einer vordefinierten Maximaltemperatur Tmax liegt. Solange dies nicht der Falls ist, erfolgt ein Rücksprung zu Schritt 200. Wenn die Temperatur die vorgegebene Maximaltemperatur Tmax überschreitet, so veranlasst die zentrale Steuereinrichtung 40 die Durchführung eines oder mehrerer der Schritte 204a-d.

Im Schritt 204a erfolgt eine Kühlung des Trinkwassers aus dem Kaltwasserstrang 16 über die Kühlstrecke 86, Hierzu kann die Steuereinrichtung 40 beispielsweise die Steuerelemente der Gruppe 102, das heißt die Abzweigventile 104, 106 und den Wärmetauscher 108 ansteuern, sodass das Wasser durch die Kühlstrecke 86 geleitet und dort gekühlt wird.

Im Schritt 204b wird Wasser aus dem Kaltwasserstrang 16 abgelassen, indem das Steuerelement 70 einer WC-Spülung 22, 22', 22" oder die gesonderte Spüleinheit 74 angesteuert wird.

Im Schritt 204c wird das Drosselventil 84 angesteuert, sodass das Wasser aus dem Kaltwasserstrang 16 des Unterversorgungsstrangs 8 über die Zirkulationsleitung 92 abgeführt wird, jedoch innerhalb des Trinkwasserversorgungssystems 2 verbleibt.

Im Schritt 204d verursacht die Steuereinrichtung 40 die Ausgabe einer Nutzermeldung. Beispielsweise kann eine für den sicheren Betrieb der Trinkwasserversorgungsanlage 2 verantwortliche Person auf ein erhöhtes Verkeimungsrisiko durch die zu hohe Kaltwassertemperatur hingewiesen werden.

Figur 10 zeigt ein Ausführungsbeispiel des Verfahrens zur Überwachung und Regelung der Warmwassertemperatur.

Bei dem Verfahren empfängt die zentrale Steuereinrichtung 40 im ersten Schritt 220 Temperaturwerte von Sensoren in einem Warmwasserstrang, beispielsweise vom Sensor 24 oder den Temperatursensoren 28 im Warmwasserstrang 14. Im zweiten Schritt 222 wird überprüft, ob die Temperatur des Wassers in dem Warmwasserstrang unterhalb eine vorgegebenen Mindesttemperatur Tmin gesunken ist. Ist dies nicht der Fall, erfolgt ein Rücksprung zu Schritt 220. Wenn die Wassertemperatur unter die Mindesttemperatur Tmin sinkt, so veranlasst die zentrale Steuereinrichtung 40 die Durchführung eines oder mehrerer der Schritte 224a-d.

Im Schritt 224a wird eine vorgesehene Heizeinrichtung, zum Beispiel die dezentrale Warmwassertherme 176, angesteuert, um das Wasser aus dem Warmwasserstrang aufzuheizen.

Im Schritt 224b wird die Spüleinheit 72 angesteuert, um Wasser aus dem Warmwasserstrang auszulassen. Im Schritt 224c wird das Drosselventil 82 angesteuert, um das Wasser aus dem Warmwasserstrang 14 über die Zirkulationsleitung 90 abzuführen. Auf diese Weise kann das Wasser im betroffenen Leitungsabschnitt ausgetauscht werden, bevor es weiter abkühlt.

Im Schritt 224d verursacht die Steuereinrichtung 40 die Ausgabe einer Nutzermeldung, zum Beispiel um auf ein erhöhtes Verkeimungsrisiko durch die zu geringe Warmwassertemperatur hinzuweisen.

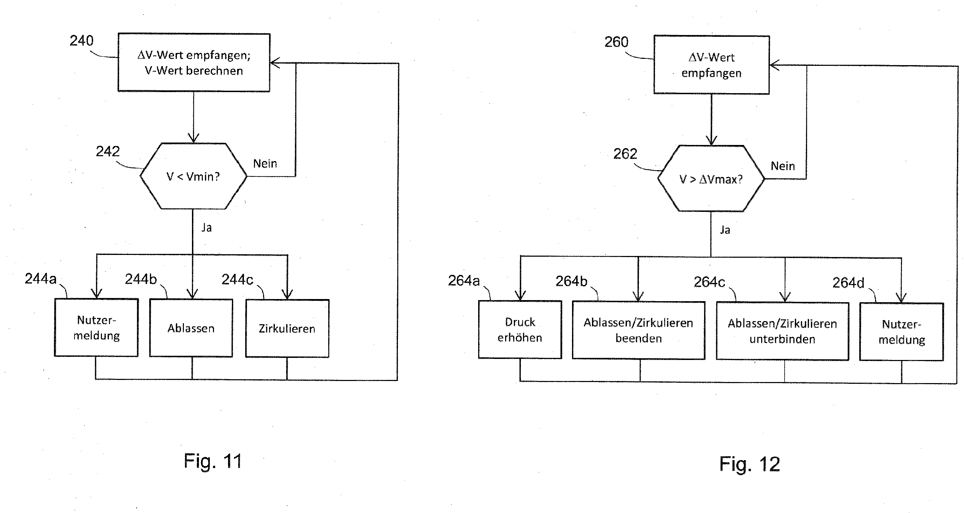

Figur 11 zeigt ein Ausführungsbeispiel des Verfahrens zur Überwachung und Regelung des Mindestdurchsatzes durch einen Trinkwasserstrang.

Bei dem Verfahren empfängt die zentrale Steuereinrichtung 40 in einem ersten Schritt den Volumenstromwert von einem Volumenstromsensor 24, 26, 30. Aus den Messwerten berechnet die Steuereinrichtung dann das Volumen des über einen vorgegebenen Zeitraum durch einen bestimmten Leitungsabschnitt eines Trinkwasserstrangs fließenden Wassers.

Im zweiten Schritt 242 wird überprüft, ob der berechnete Wasservolumenwert unterhalb eines Mindestvolumenwerts Vmin liegt. Ist dies nicht der Fall, so erfolgt ein Rücksprung zu Schritt 240. Andernfalls veranlasst die Steuereinrichtung 40 die Durchführung eines oder mehrerer der Schritte 244a-c.

Im Schritt 244a verursacht die Steuereinrichtung 40 die Ausgabe einer Nutzermeldung. Beispielsweise kann ein zu geringer Durchflusswert durch einen Trinkwasserstrang darauf hindeuten, dass eine Trinkwasserentnahmestelle defekt ist und gewartet werden muss. Durch die Ausgabe einer Nutzermeldung kann dann ein Hausmeister veranlasst werden, eine entsprechende Kontrolle durchzuführen.

Im Schritt 244b veranlasst die Steuereinrichtung 40 durch Ansteuerung der Steuerelemente 70 beziehungsweise der gesonderten Spüleinheit 72 bzw. 74 ein Spülen und damit Ablassen des Wassers aus dem entsprechenden Leitungsabschnitt des Trinkwasserstrangs, sodass durch eine künstlich herbeigeführte Spülung der Volumenstrom in dem entsprechenden Trinkwasserstrang erhöht wird. Auf diese Weise kann ein zu langes Stehen des Wassers in dem Trinkwasserstrang und damit eine Verkeimung des Wassers verhindert werden.

Im Schritt 244c veranlasst die Steuereinrichtung 40 durch Ansteuerung der Drosselventile 82 bzw. 84, dass Wasser aus den Trinkwassersträngen 14, 16 über die Zirkulationsleitungen 90, 92 abgeführt wird, und erhöht damit ebenfalls künstlich den Volumenstrom.

Figur 12 zeigt ein Ausführungsbeispiel des Verfahrens zur Überwachung und nutzungsabhängigen Regelung. Bei dem Verfahren empfängt die zentrale Steuereinrichtung 40 im ersten Schritt 260 Messwerte für den Volumenstrom, beispielsweise von den Sensoren 24, 26 oder 30.

Im Schritt 262 wird überprüft, ob der gemessene Volumenstrom oberhalb eines maximalen vorgegebenen Volumenstroms ΔVmax liegt. Ist dies nicht der Falls, erfolgt ein Rücksprung zu Schritt 260. Wenn der maximal zulässige Volumenstrom überschritten wird, kann dies darauf hinweisen, dass der entsprechende Trinkwasserstrang temporär überlastet wird, da an zu vielen Stellen gleichzeitig Wasser entnommen wird. Als Gegenmaßnahme kann die Steuereinrichtung dann einen oder mehrere der Schritte 264a-d veranlassen.

Im Schritt 264a bewirkt die Steuereinrichtung eine Wasserdruckerhöhung, beispielsweise durch eine Leistungserhöhung der Pumpe 76 bzw. 78 oder durch Öffnen eines vorgesehenen Zuleitungsventils, um für den betreffenden Trinkwasserstrang mehr Wasser bzw. einen höheren Druck zur Verfügung zu stellen.

Im Schritt 264b veranlasst die Steuereinrichtung, dass ein gegebenenfalls automatisch durchgeführter Vorgang, bei dem Wasser zum Beispiel über die Spüleinheit 72, 74 abgelassen oder über die Zirkulationsleitungen 90, 92 abgeführt wird, beendet wird. Auf diese Weise steht das ansonsten automatisch abgelassene Wasser den übrigen Trinkwasserentnahmestellen zur Verfügung.

Im Schritt 264c veranlasst die Steuereinrichtung 40, dass für einen bestimmten Zeitraum oder während der zulässige Volumenstrom überschritten ist, ein automatisches Ablassen oder Zirkulieren des Wassers unterbunden wird. Auf diese Weise wird die Versorgungssicherheit an den einzelnen Trinkwasserentnahmestellen sichergestellt.

Im Schritt 264d veranlasst die Steuereinrichtung 40 die Ausgabe einer Nutzermeldung. Beispielsweise kann eine verantwortliche Person auf einen möglichen Versorgungsengpass im entsprechenden Trinkwasserstrang hingewiesen werden.

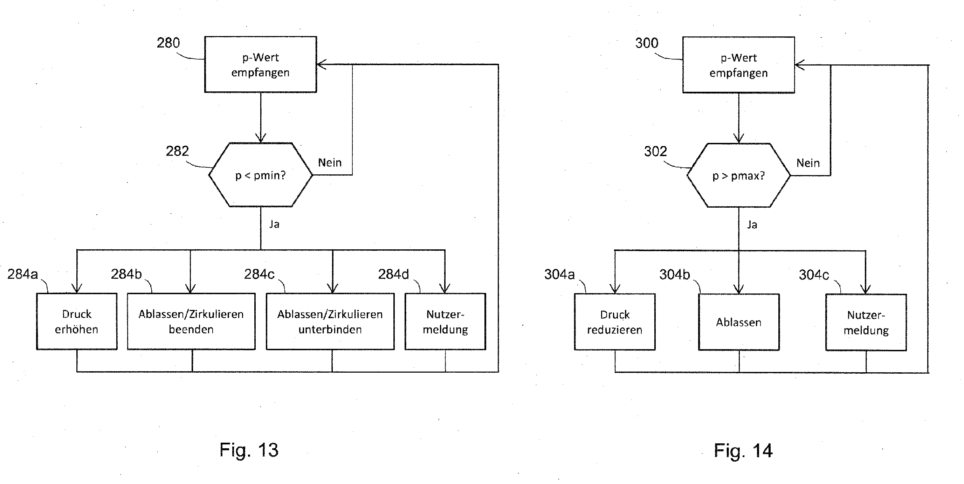

Figur 13 zeigt ein Ausführungsbeispiel des Verfahrens zur Überwachung und Regelung des Leitungsdrucks. Bei dem Verfahren empfängt die zentrale Steuereinrichtung 40 im ersten Schritt 280 einen Messwert für den Wasserdruck in dem Trinkwasserstrang, beispielsweise von Sensor 24 oder 26.

Im zweiten Schritt 282 überprüft die Steuereinrichtung 40, ob der gemessene Druck unterhalb eines Minimaldrucks pmin liegt. Ist dies nicht der Fall, erfolgt ein Rücksprung in den ersten Schritt 280. Andernfalls veranlasst die Steuereinrichtung einen oder mehrere der Schritte 284a-d.

Im Schritt 284a veranlasst die Steuereinrichtung 40 eine Wasserdruckerhöhung, beispielsweise indem die Leistung der Pumpen 76 bzw. 78 erhöht wird oder indem ein Zuleitungsventil geöffnet wird, um den Druck in den Leitungen zu erhöhen.

In den Schritten 284b-c werden ein etwaig laufender Spül- beziehungsweise Zirkulationsvorgang beendet beziehungsweise zukünftige Spül- beziehungsweise Zirkulationsvorgänge unterbunden.

Im Schritt 284d verursacht die Steuereinrichtung 40 die Ausgabe einer Nutzermeldung, beispielsweise um auf die Möglichkeit eines Lecks hinzuweisen, das ebenfalls Ursache für einen Druckabfall sein kann. Beispielsweise kann die Steuereinrichtung 40 dazu eingerichtet sein, den Druck innerhalb eines Leitungsabschnitts über einen längeren Zeitraum zu überwachen und bei einem untypischen Druckabfall oder einem Druckabfall, der über übliche Schwankungen hinausgeht, die Gefahr eines möglichen Lecks anzuzeigen.

Figur 14 zeigt ein weiteres Ausführungsbeispiel des Verfahrens zur Überwachung und Regelung des Leitungsdrucks. Bei dem Verfahren empfängt die zentrale Steuereinrichtung 40 im ersten Schritt 300 einen Wert für den Wasserdruck in dem betreffenden Trinkwasserstrang, beispielsweise von dem Sensor 24 oder 26.

Im Schritt 302 wird überprüft, ob der gemessene Druckwert oberhalb eines vorgegebenen Maximaldrucks pmax liegt. Ist dies nicht der Fall, erfolgt ein Rücksprung zum ersten Schritt 300. Andernfalls bewirkt die Steuereinrichtung die Durchführung eines oder mehrerer der Schritte 304a-c.

Im Schritt 304a wird der Wasserdruck reduziert, beispielsweise indem die Pumpleistung der Pumpe 76 bzw. 78 reduziert oder ein Zuleitungsventil geschlossen wird, um den Druck in dem betreffenden Trinkwasserstrang zu reduzieren. Alternativ oder zusätzlich kann die Steuereinrichtung 40 auch das Öffnen eines Ventils, zum Beispiel an einer Spüleinheit, veranlassen.

Im Schritt 304b wird, beispielsweise durch Ansteuerung der Spüleinheit 72 bzw. 74, Wasser aus dem betreffenden Trinkwasserstrang abgelassen, um den Wasserdruck in dem betreffenden Trinkwasserstrang zu reduzieren.

Im Schritt 304c verursacht die Steuereinrichtung 40 die Ausgabe einer Nutzermeldung, beispielsweise um auf einen kritischen Überdruck im Leitungssystems hinzuweisen.

Durch die automatische Überwachung und Regelung des Wasserdrucks im Trinkwasserleitungssystem 4 entsprechend der Fig. 13 und 14 kann weiterhin ein automatischer Druckabgleich erreicht werden. Beispielsweise können mehrere Drucksensoren sowie mehrere Pumpen und/oder Zuleitungsventile auf verschiedenen Etagen eines Gebäudekomplexes vorgesehen sein, in dem das Trinkwasserleitungssystem installiert wird. Durch eine zentrale Überwachung des Wasserdrucks auf den einzelnen Etagen und eine davon abhängige automatische Ansteuerung der Pumpen und/oder Zuleitungsventile kann der Wasserdruck auf allen Etagen in einen vorgegebenen Druckbereich geregelt werden.

Weiterhin kann hierdurch auch ein nutzungsabhängiger Druckabgleich erreicht werden, da der Wasserdruck zum Beispiel bei erhöhter Nachfrage an mehreren Trinkwasserentnahmestellen in einer Etage automatisch nachgeregelt wird.

## Patentansprüche

1. Trinkwasserversorgungssystem (2)
- mit einem Trinkwasserleitungssystem (4),
- mit mehreren an das Trinkwasserleitungssystem (4) angeschlossenen Trinkwasserentnahmestellen (18, 20, 22, 22', 22", 170), wobei das Trinkwasserleitungssystem (2) einen Trinkwasserstrang (14, 16) zur Trinkwasserversorgung mehrerer der Trinkwasserentnahmestellen (18, 20, 22, 22', 22", 170) aufweist,
- mit mindestens einem Sensor (24, 26), der dazu eingerichtet ist, Messwerte zu bestimmen, wobei der Sensor ein Drucksensor (24, 26) ist, um den Wasserdruck im Trinkwasserstrang (14,16) zu messen, und
- mit einer zentralen Steuereinrichtung (40), die dazu eingerichtet ist, die von dem mindestens einen Sensor (24, 26) bestimmten Messwerte zu empfangen und auszuwerten,
wobei
die zentrale Steuereinrichtung (40) dazu eingerichtet ist, eine der folgenden Maßnahmen zu bewirken, wenn der vom Drucksensor (24, 26) gemessene Wasserdruck einen vorgegebenen Grenzwert (pmin) über einen bestimmten Zeitraum unterschreitet:
- Beenden und/oder Unterbinden eines von der zentralen Steuereinrichtung (40) bewirkten Ablassens von Wasser aus dem Trinkwasserstrang (14, 16) an einer Spüleinheit (72, 74) oder
- Beenden und/oder Unterbinden eines von der zentralen Steuereinrichtung (40) bewirkten Abführens von Wasser aus dem Trinkwasserstrang (14, 16) über eine Zirkulationsleitung (90, 92).

2. Trinkwasserversorgungssystem nach Anspruch 1,
**dadurch gekennzeichnet,**
- **dass** mehrere Sensoren (24, 26, 28, 30, 158, 168) vorgesehen sind, die dazu eingerichtet sind, an verschiedenen Stellen im Trinkwasserversorgungssystem (2) Messwerte für eine oder verschiedene Eigenschaften des im Trinkwasserversorgungssystem (2) geführten Wassers zu bestimmen, und
- **dass** die zentrale Steuereinrichtung (40) dazu eingerichtet ist, die von den Sensoren (24, 26, 28, 30, 158, 168) bestimmten Messwerte zu empfangen und auszuwerten.

3. Trinkwasserversorgungssystem nach Anspruch 2,
**dadurch gekennzeichnet, dass** ein oder mehrere der Sensoren (24, 26, 28, 30) dazu eingerichtet sind, Messwerte für die Wassertemperatur, den Wasserdruck, den Wasserdurchfluss und/oder die Geschwindigkeitsverteilung des im Trinkwasserversorgungssystem (2) geführten Wassers zu bestimmen.

4. Trinkwasserversorgungssystem nach einem der Ansprüche 1 bis 3,
**dadurch gekennzeichnet, dass** die Steuereinrichtung (40) dazu eingerichtet ist, die Ausgabe einer von den empfangenen Messwerten abhängigen Nutzerinformation über eine Nutzerschnittstelle (52) zu bewirken.

5. Trinkwasserversorgungssystem nach einem der Ansprüche 1 bis 4,
- mit mehreren dezentralen Steuerelementen (70, 76, 78, 5 82, 84, 86, 86', 104, 106, 108, 130, 150, 160, 172, 176), die dazu eingerichtet sind, an verschiedenen Stellen im Trinkwasserversorgungssystem (2) eine oder mehrere Eigenschaften des im Trinkwasserversorgungssystem (2) geführten Wassers zu beeinflussen,
- wobei die zentrale Steuereinrichtung (40) dazu eingerichtet ist, die Steuerelemente (70, 76, 78, 82, 84, 86, 86`,104,106, 108,130, 150, 160, 172, 176) zur Beeinflussung der einen oder mehreren Eigenschaften des im Trinkwasserversorgungssystem (2) geführten Wassers anzusteuern, und
- wobei die zentrale Steuereinrichtung (40) dazu eingerichtet ist, die Steuerelemente (70, 76, 78, 82, 84, 86, 86', 104, 106, 108, 130, 150, 160, 172, 176) abhängig von den empfangenen Messwerten zu steuern.

6. Trinkwasserversorgungssystem nach Anspruch 5,
**dadurch gekennzeichnet,**
- **dass** ein oder mehrere der dezentralen Steuerelemente (70, 76, 78, 82, 84, 86, 86', 104, 106, 108, 130, 160, 172, 176) dazu eingerichtet sind, die Wassertemperatur, den Wasserdruck, den Wasserdurchfluss und/oder die Geschwindigkeitsverteilung des im Trinkwasserversorgungssystem (2) geführten Wassers zu beeinflussen, und
- **dass** die zentrale Steuereinrichtung (40) dazu eingerichtet ist, die ein oder mehreren der dezentralen Steuerelemente (70, 76, 78, 82, 84, 86, 86', 104, 106, 108, 130, 160, 172, 176) zur Beeinflussung der Wassertemperatur, des Wasserdrucks, des Wasserdurchflusses und/oder der Geschwindigkeitsverteilung des im Trinkwasserversorgungssystem (2) geführten Wassers anzusteuern.

7. Trinkwasserversorgungssystem nach einem der Ansprüche 1 bis 6,
**dadurch gekennzeichnet,**
- **dass** in den Trinkwasserstrang (10, 12, 14, 16) eine von der zentralen Steuereinrichtung (40) ansteuerbare gesonderte Spüleinheit (74) integriert ist, durch die Wasser aus dem Trinkwasserversorgungssystem (2) abgelassen werden kann.

8. Trinkwasserversorgungssystem nach einem der Ansprüche 1 bis 7,
**dadurch gekennzeichnet, dass** eine von der zentralen Steuereinrichtung (40) ansteuerbare dezentrale Steuereinheit (70) vorgesehen ist, die dazu eingerichtet ist, einen Spülvorgang an einer Trinkwasserentnahmestelle (18, 20, 22, 22', 22", 170) zu bewirken, um Wasser aus dem Trinkwasserversorgungssystem (2) abzulassen.

9. Trinkwasserversorgungssystem nach einem der Ansprüche 1 bis 8,
**dadurch gekennzeichnet, dass**
- dass die Steuereinrichtung (40) dazu eingerichtet ist, eine der folgenden Maßnahmen zu bewirken, wenn der vom Drucksensor (24, 26) gemessene Wasserdruck einen vorgegebenen Grenzwert (pmax) überschreitet:
- Reduzierung des Wasserdrucks im Trinkwasserstrang (14, 16), insbesondere durch Deaktivierung oder Leistungsreduzierung einer im Trinkwasserversorgungssystem (2) vorgesehenen Wasserpumpe (76, 78),
- Ablassen des Wassers aus dem Trinkwasserstrang (14, 16) an einer Spüleinheit (72, 74) oder
- Ausgabe einer Nutzermeldung.

10. Verfahren zur Steuerung eines Trinkwasserversorgungssystems (2) nach einem der Ansprüche 1 bis 9, umfassend folgende Schritte:
- Empfangen von Messwerten, insbesondere von Messwerten für eine oder verschiedene Eigenschaften des im Trinkwasserversorgungssystem (2) geführten Wassers, und
- Steuern des Trinkwasserversorgungssystems (2) abhängig von den empfangenen Messwerten,
**dadurch gekennzeichnet,**
- **dass** von der zentralen Steuereinrichtung (40) des Trinkwasserversorgungssystems (2) eine der folgenden Maßnahmen bewirkt wird, wenn ein von einem Drucksensor des Trinkwasserversorgungssystems (2) gemessener Wasserdruck einen vorgegebenen Grenzwert (pmin) über einen bestimmten Zeitraum unterschreitet:
- Beenden und/oder Unterbinden eines von der zentralen Steuereinrichtung (40) bewirkten Ablassens von Wasser aus dem Trinkwasserstrangs (14, 16) an einer Spüleinheit (72, 74) oder
- Beenden und/oder Unterbinden eines von der zentralen Steuereinrichtung (40) bewirkten Abführens von Wassers aus dem Trinkwasserstrang (14, 16) über eine Zirkulationsleitung (90, 92).

11. Computerprogramm aufweisend Befehle deren Ausführung auf mindestens einem Prozessor einer Steuereinrichtung eines Trinkwasserversorgungssystems (2) nach einem der Ansprüche 1 bis 9 die Durchführung eines Verfahrens nach Anspruch 10 bewirkt.

## Claims

1. Drinking water supply system (2)
- with a drinking water pipe system (4),
- with a plurality of drinking water tapping points (18, 20, 22, 22', 22", 170) connected to the drinking water pipe system (4), wherein the drinking water pipe system (2) has a drinking water line (14, 16) for the supply of drinking water to a plurality of the drinking water tapping points (18, 20, 22, 22', 22", 170),
- with at least one sensor (24, 26) configured to determine measurement values, the sensor being a pressure sensor (24, 26) for measuring the water pressure in the drinking water line (14, 16), and
- with a central control device (40) configured to receive and analyse the measurement values determined by the at least one sensor (24, 26),
- wherein the central control device (40) is configured to effect one of the following measures if the water pressure measured by the pressure sensor (24, 26) falls below a predetermined limit value (pmin) over a certain period of time:
- ending and/or preventing a draining of water, effected by the central control device (40), from the drinking water line (14, 16) at a flushing unit (72, 74), or
- ending and/or preventing a discharging of water, effected by the central control device (40), from the drinking water line (14, 16) via a circulation line (90, 92).

2. Drinking water supply system according to claim 1,
**characterised**
- **in that** a plurality of sensors (24, 26, 28, 30, 158, 168) are provided, which are configured to determine measurement values for one or various properties of the water carried in the drinking water supply system (2) at different points in the drinking water supply system (2), and
- **in that** the central control device (40) is configured to receive and analyse the measurement values determined by the sensors (24, 26, 28, 30, 158, 168).

3. Drinking water supply system according to claim 2,
**characterised in that** one or more of the sensors (24, 26, 28, 30) are configured to determine measurement values for the water temperature, the water pressure, the water flow rate and/or the velocity distribution of the water carried in the drinking water supply system (2).

4. Drinking water supply system according to one of claims 1 to 3,
**characterised in that** the control device (40) is configured to effect the output of user information dependent on the received measurement values via a user interface (52).

5. Drinking water supply system according to any one of claims 1 to 4,
- comprising a plurality of decentralised control elements (70, 76, 78, 5 82, 84, 86, 86', 104, 106, 108, 130, 150, 160, 172, 176), which are configured to influence one or more properties of the water carried in the drinking water supply system (2) at different points in the drinking water supply system (2),
- wherein the central control device (40) is configured to actuate the control elements (70, 76, 78, 82, 84, 86, 86', 104, 106, 108, 130, 150, 160, 172, 176) to influence the one or more properties of the water carried in the drinking water supply system (2), and
- wherein the central control device (40) is configured to control the control elements (70, 76, 78, 82, 84, 86, 86', 104, 106, 108, 130, 150, 160, 172, 176) depending on the measurement values received.

6. Drinking water supply system according to claim 5,
**characterised**
- **in that** one or more of the decentralised control elements (70, 76, 78, 82, 84, 86, 86', 104, 106, 108, 130, 160, 172, 176) are configured to influence the water temperature, the water pressure, the water flow rate and/or the velocity distribution of the water carried in the drinking water supply system (2), and
- **in that** the central control device (40) is configured to control the one or more of the decentralised control elements (70, 76, 78, 82, 84, 86, 86', 104, 106, 108, 130, 160, 172, 176) to influence the water temperature, the water pressure, the water flow rate and/or the velocity distribution of the water carried in the drinking water supply system (2).

7. Drinking water supply system according to one of claims 1 to 6,
**characterised in that**,
- that a separate flushing unit (74), which can be controlled by the central control device (40) and through which water can be drained from the drinking water supply system (2), is integrated into the drinking water line (10, 12, 14, 16).

8. Drinking water supply system according to one of claims 1 to 7,
**characterised in that** a decentralised control unit (70) is provided which can be controlled by the central control device (40) and which is configured to effect a flushing process at a drinking water tapping point (18, 20, 22, 22', 22", 170) in order to drain water from the drinking water supply system (2).

9. Drinking water supply system according to one of claims 1 to 8,
**characterised**
- **in that** the control device (40) is configured to effect one of the following measures if the water pressure measured by the pressure sensor (24, 26) exceeds a predetermined limit value (pmax):
- reducing the water pressure in the drinking water line (14, 16), in particular by deactivating or reducing the output of a water pump (76, 78) provided in the drinking water supply system (2),
- draining the water from the drinking water line (14, 16) at a flushing unit (72, 74) or
- output of a user message.

10. Method for controlling a drinking water supply system (2) according to any one of claims 1 to 9, comprising the following steps:
- receiving measurement values, in particular measurement values for one or more properties of the water carried in the drinking water supply system (2), and
- controling the drinking water supply system (2) depending on the measurement values received,
**characterised**
- **in that** one of the following measures is effected by the central control device (40) of the drinking water supply system (2) if a water pressure measured by a pressure sensor of the drinking water supply system (2) falls below a predetermined limit value (pmin) over a certain period of time:
- ending and/or preventing a draining of water, effected by the central control device (40), from the drinking water line (14, 16) at a flushing unit (72, 74), or
- ending and/or preventing a discharging of water, effected by the central control device (40), from the drinking water line (14, 16) via a circulation line (90, 92).

11. Computer program comprising instructions the execution of which on at least one processor of a control device of a drinking water supply system (2) according to one of claims 1 to 9 causes the execution of a method according to claim 10.

## Revendications

1. Système d'approvisionnement en eau potable (2)
- avec un système de conduite d'eau potable (4),
- avec plusieurs points de prélèvement d'eau potable (18, 20, 22, 22', 22", 170) raccordés au système de conduite d'eau potable (4), le système de conduite d'eau potable (2) présentant une ligne d'eau potable (14, 16) pour l'alimentation en eau potable de plusieurs des points de prélèvement d'eau potable (18, 20, 22, 22', 22", 170),
- avec au moins un capteur (24, 26) qui est configuré pour déterminer des valeurs de mesure, le capteur étant un capteur de pression (24, 26) pour mesurer la pression de l'eau dans la ligne d'eau potable (14, 16), et
- avec un dispositif de commande central (40), qui est configuré pour recevoir et évaluer les valeurs de mesure déterminées par l'au moins un capteur (24, 26),
- où le dispositif de commande central (40) est configuré pour provoquer l'une des mesures suivantes lorsque la pression de l'eau mesurée par le capteur de pression (24, 26) est inférieure à une valeur limite prédéfinie (pmin) pendant une durée déterminée :
- terminer et/ou empêcher une évacuation d'eau de la ligne d'eau potable (14, 16) provoquée par le dispositif de commande central (40) au niveau d'une unité de rinçage (72, 74) ou
- terminer et/ou empêcher une évacuation d'eau de la ligne d'eau potable (14, 16) via une conduite de circulation (90, 92) provoquée par le dispositif de commande central (40).

2. Système d'alimentation en eau potable selon la revendication 1, **caractérisé**
- **en ce que** plusieurs capteurs (24, 26, 28, 30, 158, 168) sont prévus, lesquels plusieurs capteurs sont configurés pour déterminer, à différents endroits dans le système d'alimentation en eau potable (2), des valeurs de mesure pour une ou différentes propriétés de l'eau acheminée dans le système d'alimentation en eau potable (2), et
- **en ce que** le dispositif de commande central (40) est configuré pour recevoir et évaluer les valeurs de mesure déterminées par les capteurs (24, 26, 28, 30, 158, 168).

3. Système d'alimentation en eau potable selon la revendication 2, **caractérisé en ce qu'**un ou plusieurs des capteurs (24, 26, 28, 30) sont configurés pour déterminer des valeurs de mesure pour la température de l'eau, la pression de l'eau, le débit de l'eau et/ou la répartition de la vitesse de l'eau circulant dans le système d'alimentation en eau potable (2).

4. système d'alimentation en eau potable selon l'une des revendications 1 à 3, **caractérisé en ce que** le dispositif de commande (40) est adapté pour provoquer l'émission, par l'intermédiaire d'une interface utilisateur (52), d'une information d'utilisateur dépendant des valeurs de mesure reçues.

5. Système d'alimentation en eau potable selon l'une des revendications 1 à 4,
- avec plusieurs éléments de commande décentralisés (70, 76, 78, 5 82, 84, 86, 86', 104, 106, 108, 130, 150, 160, 172, 176) qui sont configurés pour influencer, à différents endroits dans le système d'alimentation en eau potable (2), une ou plusieurs propriétés de l'eau acheminée dans le système d'alimentation en eau potable (2),
- le dispositif de commande central (40) étant configuré pour commander les éléments de commande (70, 76, 78, 82, 84, 86, 86', 104, 106, 108, 130, 150, 160, 172, 176) pour influencer l'une ou plusieurs propriétés de l'eau acheminée dans le système d'alimentation en eau potable (2), et
- le dispositif de commande central (40) étant configuré pour commander les éléments de commande (70, 76, 78, 82, 84, 86, 86', 104, 106, 108, 130, 150, 160, 172, 176) en fonction des valeurs de mesure reçues.

6. Système d'alimentation en eau potable selon la revendication 5,
**caractérisé**
- **en ce qu'**un ou plusieurs des éléments de commande décentralisés (70, 76, 78, 82, 84, 86, 86', 104, 106, 108, 130, 160, 172, 176) sont configurés pour influencer la température de l'eau, la pression de l'eau, le débit de l'eau et/ou la répartition de la vitesse de l'eau circulant dans le système d'alimentation en eau potable (2), et
- **en ce que** le dispositif de commande central (40) est configuré pour commander un ou plusieurs des éléments de commande décentralisés (70, 76, 78, 82, 84, 86, 86', 104, 106, 108, 130, 160, 172, 176) pour influencer la température de l'eau, la pression de l'eau, le débit de l'eau et/ou la répartition de la vitesse de l'eau acheminée dans le système d'alimentation en eau potable (2).

7. Système d'alimentation en eau potable selon l'une des revendications 1 à 6,
**caractérisé**
- **en ce qu'**une unité de rinçage (74) séparée, pouvant être commandée par le dispositif de commande central (40), est intégrée dans la ligne d'eau potable (10, 12, 14, 16), par laquelle de l'eau peut être évacuée du système d'alimentation en eau potable (2).

8. Système d'alimentation en eau potable selon l'une des revendications 1 à 7, **caractérisé en ce qu'**une unité de commande décentralisée (70) pouvant être commandée par le dispositif de commande central (40) est prévue, laquelle unité de commande décentralisée est configurée pour provoquer une opération de rinçage à un point de prélèvement d'eau potable (18, 20, 22, 22', 22", 170) afin d'évacuer de l'eau du système d'alimentation en eau potable (2).

9. Système d'alimentation en eau potable selon l'une des revendications 1 à 8, **caractérisé**
- **en ce que** le dispositif de commande (40) est configuré pour provoquer l'une des mesures suivantes lorsque la pression de l'eau mesurée par le capteur de pression (24, 26) dépasse une valeur limite prédéterminée (pmax) :
- réduction de la pression de l'eau dans la ligne d'eau potable (14, 16), en particulier par désactivation ou réduction de la puissance d'une pompe à eau (76, 78) prévue dans le système d'alimentation en eau potable (2),
- évacuer l'eau de la ligne d'eau potable (14, 16) à une unité de rinçage (72, 74) ou
- émettre un message utilisateur.

10. Procédé de commande d'un système d'alimentation en eau potable (2) selon l'une des revendications 1 à 9, comprenant les étapes suivantes :
- recevoir des valeurs de mesure, en particulier de valeurs de mesure pour une ou différentes propriétés de l'eau acheminée dans le système d'alimentation en eau potable (2), et
- commander le système d'alimentation en eau potable (2) en fonction des valeurs de mesure reçues,
**caractérisé**
- **en ce que** le dispositif de commande central (40) du système d'alimentation en eau potable (2) provoque une des mesures suivantes lorsqu'une pression d'eau mesurée par un capteur de pression du système d'alimentation en eau potable (2) est inférieure à une valeur limite prédéfinie (pmin) pendant une durée déterminée :
- terminer et/ou empêcher une évacuation d'eau de la ligne d'eau potable (14, 16) provoquée par le dispositif de commande central (40) au niveau d'une unité de rinçage (72, 74) ou
- terminer et/ou empêcher une évacuation d'eau de la ligne d'eau potable (14, 16) via une conduite de circulation (90, 92) provoquée par le dispositif de commande central (40).

11. Programme informatique comportant des instructions dont l'exécution sur au moins un processeur d'un dispositif de commande d'un système d'alimentation en eau potable (2) selon l'une des revendications 1 à 9 provoque la mise en oeuvre d'un procédé selon la revendication 10.
